(19) Europäisches Patentamt
European Patent Office
Office européen des brevets

(11) EP 1 578 415 B1

(12) **EUROPEAN PATENT SPECIFICATION**

(45) Date of publication and mention of the grant of the patent:
**10.09.2008 Bulletin 2008/37**

(21) Application number: **03775750.7**

(22) Date of filing: **09.12.2003**

(51) Int Cl.:
***A61K 31/40*** *(2006.01)* ***A61K 47/38*** *(2006.01)*
***A61K 9/16*** *(2006.01)* ***A61K 31/4706*** *(2006.01)*

(86) International application number:
**PCT/IB2003/005861**

(87) International publication number:
**WO 2004/056358 (08.07.2004 Gazette 2004/28)**

(54) **DOSAGE FORMS COMPRISING A CETP INHIBITOR AND AN HMG-COA REDUCTASE INHIBITOR**

DOSIERUNGSFORM ENTHALTEND EINEN CETP-HEMMER UND EINEN HMG-COA REDUKTASE HEMMER

FORMES POSOLOGIQUES COMPRENANT UN INHIBITEUR DE CETP ET UN INHIBITEUR DE HMG-COA REDUCTASE

(84) Designated Contracting States:
**AT BE BG CH CY CZ DE DK EE ES FI FR GB GR HU IE IT LI LU MC NL PT RO SE SI SK TR**

(30) Priority: **20.12.2002 US 435298 P**

(43) Date of publication of application:
**28.09.2005 Bulletin 2005/39**

(60) Divisional application:
**08157637.3 / 1 961 419**

(73) Proprietor: **Pfizer Products Inc.**
**Groton, CT 06340 (US)**

(72) Inventors:
- **FRIESEN, Dwayne, Thomas**
  **Bend, OR 97702 (US)**
- **LYON, David, Keith**
  **Bend, OR 97702 (US)**
- **LORENZ, Douglas, Alan**
  **Bend, OR 97702 (US)**
- **HANCOCK, Bruno, Caspar,**
  **Pfizer Global R.& D.**
  **Groton, CT 06340 (US)**
- **KETNER, Rodney, James**
  **Bend, OR 97701 (US)**
- **MCDERMOTT, Timothy, Joseph,**
  **Pfizer Global R. & D.**
  **Groton, CT 06340 (US)**
- **SHANKER, Ravi, Mysore,**
  **Pfizer Global R. & D.**
  **Groton, CT 06340 (US)**

(74) Representative: **Ruddock, Keith Stephen**
**Pfizer Limited,**
**European Pharma Patent Department,**
**Ramsgate Road**
**Sandwich,**
**Kent CT13 9NJ (GB)**

(56) References cited:

| | |
|---|---|
| **WO-A-00/38722** | **WO-A-02/11710** |
| **WO-A-02/13797** | **WO-A-02/100394** |
| **WO-A-03/000292** | **US-B1- 6 310 075** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Background

The present invention relates to a dosage form comprising: (1) a solid amorphous dispersion comprising a cholesteryl ester transfer protein (CETP) inhibitor and a concentration-enhancing polymer; and (2) an add-sensitive HMG-CoA reductase inhibitor.

It is well known that inhibitors of 3-hydroxy-3-methylglutaryl-coenzyme A reductase (HMG-CoA reductase), an important enzyme catalyzing the Intracellular synthesis of cholesterol, will bring about reduced levels of blood cholesterol, especially In terms of the low density lipoprotein (LDL) form of cholesterol. Therefore, HMG-CoA reductase enzyme inhibitors are considered potentially useful as hypocholesterolemic or hypolipidemic agents.

CETP inhibitors are another class of compounds that are capable of modulating levels of blood cholesterol such as by raising high density lipoprotein (HDL) cholesterol and lowering LDL cholesterol. CETP inhibitors have extremely low aqueous solutility. Accordingly, CETP inhibitors must be formulated so as to be capable of providing good bioavailability. One method for increasing the bioavailability of a CETP inhibitor is to form a solid amorphous dispersion of the drug and a concentration-enhancing polymer. See, e.g., WO02/11710 A2.

It Is well known that a combination therapy of a CETP Inhibitor and an HMG-CoA reductase inhibitor may be used to treat elevated LDL cholesterol and low HDL cholesterol levels. For example, WO02/13797 A2 relates to pharmaceutical combinations of cholesteryl ester transfer protein inhibitors and atorvastatin. The application discloses that the compounds may be generally administered separately or together, with a pharmaceutically acceptable carrier, vehicle or diluent. The compounds may be administered individually or together in any conventional oral, parenteral or transdermal dosage form. For oral administration, the composition may take the form of solutions, suspensions, tablets, pills, capsules, powders and the like.

DeNinno et al., U.S. Patent 6,310,075 B1 relates to CETP inhibitors, pharmaceutical compositions containing such inhibitors and the use of such inhibitors. DeNinno et al. disclose a pharmaceutical combination composition comprising a CETP inhibitor and an HMG-CoA reductase inhibitor. DeNinno disclose that the compounds of the invention may be administered in the form of a pharmaceutical composition comprising at least one of the compounds, together with a pharmaceutically acceptable vehicle, diluent, or carrier. For oral administration a pharmaceutical composition can take the form of solutions, suspensions, tablets, pills, capsules, powders and the like. Similarly, DeNinno et al., U.S. Patent No. 6,197,786 B1 disclose pharmaceutical combinations comprising CETP inhibitors and HMG-CoA reductase inhibitors.

WO 00/38722 discloses combinations of CETP inhibitors and HMG-CoA reductase inhibitors for cardiovascular indications. The pharmaceutical compositions include those suitable for oral, rectal, topical, buccal, and parenteral administration. The application discloses solid dosage forms for oral administration including capsules, tablets, pills, powders, gel caps and granules.

Schmeck et al., U.S. Patent No. 5,932,587 disclose another class of CETP inhibitors. Schmeck et al. disclose that the CETP inhibitors may be used in combination with certain HMG-CoA reductase inhibitors such as statins, including atorvastatin.

However, while it is desired to combine the CETP inhibitor and an HMG-CoA reductase inhibitor into a single dosage form, combining a CETP inhibitor and an HMG-CoA reductase inhibitor into a single dosage form presents a number of potential problems. Some HMG-CoA reductase inhibitor compounds are unstable in that they are susceptible to heat, moisture, low pH environment, and light. Some HMG-CoA reductase inhibitors, such as atorvastatin, pravastatin, fluvastatin, rosuvastatin, and cerivastatin are in the form of hydroxy acids that will degrade to a lactone in an acidic environment. Other HMG-CoA reductase inhibitors, such as lovastatin and simvastatin, contain substituents that readily degrade in an acidic environment. When packaged in the form of tablets, powders, granules, or within capsules, the HMG-CoA reductase inhibitor may be further destabilized by contact with the molecular moieties of other components of the dosage form. Since pharmaceutical dosage form components such as binders, diluents, antiadherents, surfactants and the like may adversely interact with the active ingredient compound, a stabilizing means may be required for effective pharmaceutical dosages. For example, U.S. Patent No. 6,126,971 discloses the addition of a stabilizing agent such as calcium carbonate to stabilize the HMG-CoA reductase inhibitor atorvastatin calcium. Nevertheless, the means for stabilizing the HMG-CoA reductase inhibitor must also allow solubilization of the CETP inhibitor.

Accordingly, what is desired is a dosage form containing a CETP inhibitor and an HMG-CoA reductase inhibitor that stabilizes the HMG-CoA reductase inhibitor and that provides good bioavailability for the CETP inhibitor.

Summary of the Invention

The present invention overcomes the drawbacks of the prior art by providing a unitary dosage form comprising (1) a solid amorphous dispersion comprising a CETP inhibitor and a concentration-enhancing polymer and (2) an HMG-CoA reductase inhibitor wherein said concentration-enhancing polymer is a neutralised acidic polymer in which at least 90% of the acidic moieties have been neutralised; and wherein the CETP inhibitor is (2R)-3-[[3-(4-chloro-3-ethylphenoxy) phenyl][[3-(1,1,2,2)-tetrafluoro ethoxy)phenyl]-methyl]amino]-1,1,1-trifluoro-2-propanol.

The concentration-enhancing polymer chosen to form the solid amorphous dispersion should be an acidic polymer wherein a sufficient quantity of the acidic groups In the polymer have been neutralized, so that the polymer does not cause adverse chemical degradation of the HMG-CoA reductase inhibitor. The HMG-CoA reductase inhibitor In the resulting unitary dosage form has improved chemical stability when compared to a control dosage form where the concentration-enhancing polymer In the control dosage form is the unneutralized acidic polymer.

By "unitary dosage form" is meant a single dosage form containing both the CETP inhibitor and HMG-CoA reductase inhibitor so that, following administration of the unitary dosage form to a use environment, both the CETP inhibitor and HMG-CoA reductase inhibitor are delivered to the use environment. The term "unitary dosage form" includes a single tablet, caplet, pill, capsule, powder, or a kit comprising one or more tablets, caplets, pills, capsules, sachets, powders, or solutions intended to be taken together.

Reference to a "use environment" can either mean *in vivo* fluids, such as the GI tract, subdermal, intranasal, buccal, intrathecal, ocular, intraaural, subcutaneous spaces, vaginal tract, arterial and venous blood vessels, pulmonary tract or intramuscular tissue of an animal, such as a mammal and particularly a human, or the *in vitro* environment of a test solution, such as phosphate buffered saline (PBS) or a Model Fasted Duodenal (MFD) solution. An appropriate PBS solution is an aqueous solution comprising 20 mM sodium phosphate ($Na_2HPO_4$), 47 mM potassium phosphate ($KH_2PO_4$), 87 mM NaCl, and 0.2 mM KCl, adjusted to pH 6.5 with NaOH. An appropriate MFD solution is the same PBS solution wherein additionally is present 7.3 mM sodium taurocholic acid and 1.4 mM of 1-palmitoyl-2-oleyl-sn-glycero-3-phosphocholine.

"Administration" to a use environment means, where the *in vivo* use environment is the GI tract, delivery by ingestion or swallowing or other such means to deliver the drugs. One skilled in the art will understand that "administration" to other *in vivo* use environments means contacting the use environment with the composition of the invention using methods known in the art. See for example, Remington: The Science and Practice of Pharmacy, 20th Edition (2000). Where the use environment is *in vitro,* "administration" refers to placement or delivery of the dosage form to the *in vitro* test medium. Where release of drug into the stomach is not desired but release of the drug in the duodenum or small intestine is desired, the use environment may also be the duodenum or small intestine. In such cases, "introduction" to a use environment is that point in time when the dosage form leaves the stomach and enters the duodenum.

The inventors have found that the bioavailability of CETP inhibitors may be substantially improved by forming a solid amorphous dispersion of the CETP inhibitor and a concentration-enhancing polymer. The administration of the CETP inhibitor in the form of a solid amorphous dispersion containing a concentration-enhancing polymer substantially increases the concentration of dissolved CETP inhibitor in the use environment relative to administration of the CETP inhibitor in crystalline form. In turn, this enhanced concentration of dissolved CETP inhibitor results In an increase in the bioavailability of the CETP inhibitor as indicated by an increase In the area under the concentration versus time curve (AUC) In the blood.

However, when an HMG-CoA reductase inhibitor is mixed directly with a solid amorphous dispersion comprising the CETP inhibitor and the acidic concentration-enhancing polymer HPMCAS and then granulated In a tableting formulation, the inventors observe chemical degradation of the HMG-CoA reductase inhibitor that is greater than that observed for the HMG-CoA reductase inhibitor alone. The inventors solved the chemical degradation problem by replacing the acidic concentration-enhancing polymer with a neutralized acidic concentration-enhancing polymer in which at least 90% of the acidic moieties have been neutralized. The inventors believe that the chemical degradation of the HMG Co-A reductase inhibitor was caused either directly by the acidic concentration-enhancing polymer or indirectly by migration of the acid to the surface of the HMG-CoA reductase inhibitor. The inventors found that the chemical stability of the HMG-CoA reductase inhibitor in the unitary dosage form could be improved by replacing the acidic polymer with a neutralized acidic polymer in which at least 90% of the acidic moieties have been neutralized. In addition, the resulting solid amorphous dispersion provides concentration enhancement in a use environment for the CETP inhibitor.

The foregoing and other objectives, features, and advantages of the invention will be more readily understood upon consideration of the following detailed description of the invention.

## Detailed Description of the Present Invention

The present invention combines a CETP inhibitor and an HMG-CoA reductase inhibitor in a unitary dosage form. The CETP inhibitor is in the form of a solid amorphous dispersion comprising a neutralized acidic concentration-enhancing polymer in which at least 90% of the acidic moieties have been neutralized. Unitary dosage forms, solid amorphous dispersions, neutralized concentration-enhancing polymers, drugs, excipients, and methods for forming the dosage forms are discussed in more detail below.

SOLID AMORPHOUS DISPERSIONS OF CETP INHIBITORS

The CETP inhibitor and concentration-enhancing polymer are combined and formed into a solid amorphous dispersion. By solid amorphous dispersion is meant a solid material In which at least a portion of the CETP inhibitor is In the amorphous form and dispersed in the polymer. Preferably, at least a major portion of the CETP inhibitor in the solid amorphous dispersion Is amorphous. By "amorphous" is meant simply that the CETP inhibitor is in a non-crystalline state. As used herein, the term "a major portion" of the CETP inhibitor means that at least 60 wt% of the CETP inhibitor in the solid amorphous dispersion is in the amorphous form, rather than the crystalline form. Preferably, the CETP inhibitor in the solid amorphous dispersion is substantially amorphous. As used herein, "substantially amorphous" means that the amount of CEPT inhibitor in crystalline form does not exceed about 25 wt%. More preferably, the CETP inhibitor in the solid amorphous dispersion is "almost completely amorphous," meaning that the amount of CETP inhibitor in the crystalline form does not exceed about 10 wt%. Amounts of crystalline CETP inhibitor may be measured by Powder X-Ray Diffraction (PXRD), Scanning Electron Microscope (SEM) analysis, differential scanning calorimetry (DSC), or any other standard quantitative measurement.

The solid amorphous dispersion may contain from about 1 to about 80 wt% CETP inhibitor, depending on the dose of the CETP inhibitor and the effectiveness of the concentration-enhancing polymer. Enhancement of aqueous CETP inhibitor concentrations and relative bioavailability are typically best at low CETP inhibitor levels, typically less than about 25 to about 40 wt%. However, due to the practical limit of the dosage form size, higher CETP inhibitor levels may be preferred and in many cases perform well.

The amorphous CETP inhibitor can exist within the solid amorphous dispersion in relatively pure amorphous drug domains or regions, as a solid solution of drug homogeneously distributed throughout the polymer or any combination of these states or those states that lie intermediate between them. The solid amorphous dispersion is preferably substantially homogeneous so that the amorphous CETP inhibitor is dispersed as homogeneously as possible throughout the polymer. As used herein, "substantially homogeneous" means that the fraction of CETP inhibitor that is present in relatively pure amorphous drug domains or regions within the solid amorphous dispersion is relatively small, on the order of less than 20 wt%, and preferably less than 10 wt% of the total amount of drug. Solid amorphous dispersions that are substantially homogeneous generally are more physically stable and have improved concentration-enhancing properties and, in turn, improved bioavailability, relative to nonhomogeneous dispersions.

In cases where the CETP inhibitor and the polymer have glass transition temperatures sufficiently far apart (greater than about 20°C), the fraction of drug that is present in relatively pure amorphous drug domains or regions within the solid amorphous dispersion can be determined by examining the glass transition temperature ($T_g$) of the solid amorphous dispersion. $T_g$ as used herein is the characteristic temperature where a glassy material, upon gradual heating, undergoes a relatively rapid (*e.g.*, in 10 to 100 seconds) physical change from a glassy state to a rubbery state. The $T_g$ of an amorphous material such as a polymer, drug, or dispersion can be measured by several techniques, including by a dynamic mechanical analyzer (DMA), a dilatometer, a dielectric analyzer, and by DSC. The exact values measured by each technique can vary somewhat, but usually fall within 10° to 30°C of each other. When the solid amorphous dispersion exhibits a single $T_g$, the amount of CETP inhibitor in pure amorphous drug domains or regions in the solid amorphous dispersion is generally has less than about 10 wt%, confirming that the solid amorphous dispersion is substantially homogeneous. This is in contrast to a simple physical mixture of pure amorphous drug particles and pure amorphous polymer particles which generally display two distinct $T_g$s, one being that of the drug and one that of the polymer. For a solid amorphous dispersion that exhibits two distinct $T_g$s, one in the proximity of the drug $T_g$ and one of the remaining drug/polymer dispersion, at least a portion of the drug is present in relatively pure amorphous domains. The amount of CETP inhibitor present in relatively pure amorphous drug domains or regions may be determined by first preparing calibration standards of substantially homogeneous dispersions to determine $T_g$ of the solid amorphous dispersion versus drug loading in the dispersion. From these calibration data and the $T_g$ of the drug/polymer dispersion, the fraction of CETP inhibitor in relatively pure amorphous drug domains or regions can be determined. Alternatively, the amount of CETP inhibitor present in relatively pure amorphous drug domains or regions may be determined by comparing the magnitude of the heat capacity for the transition in the proximity of the drug $T_g$ with calibration standards consisting essentially of a physical mixture of amorphous drug and polymer. In either case, a solid amorphous dispersion is considered to be substantially homogeneous if the fraction of CETP inhibitor that is present in relatively pure amorphous drug domains or regions within the solid amorphous dispersion is less than 20 wt%, and preferably less than 10 wt% of the total amount of CETP inhibitor.

CONCENTRATION ENHANCING POLYMERS

The concentration-enhancing polymers suitable for use in the solid amorphous dispersions of the present invention should be inert, in the sense that they do not chemically react with the CETP inhibitor in an adverse manner when present in the composition. The polymer should also have an aqueous-solubility of at least 0.1 mg/mL over at

least a portion of the pH range of 1-8. While specific polymers are discussed as being suitable for use in the compositions of the present invention, blends of such polymers may also be suitable. Thus the term "polymer" is Intended to include blends of polymers In addition to a single species of polymer.

NEUTRALIZED ACIDIC POLYMERS

Polymers suitable for use with the solid amorphous dispersions of the present invention are neutralized acidic polymers in which of least 90% of the acidic moieties have been neutralized. By "acidic polymer" is meant any polymer that possesses a significant number of acidic moieties. In general, a significant number of acidic moieties would be greater than or equal to about 0.05 milliequivalents of acidic moieties per gram of polymer. "Acidic moieties" include any functional groups that are sufficiently acidic that, In contact with or dissolved In water, can at least partially donate a hydrogen cation to water and thus increase the hydrogen-ion concentration. This definition includes any functional group or "substituent," as it is termed when the functional group is covalently attached to a polymer, that has a $pK_a$ of less than about 10. Here, the term $pK_a$ is used In its traditional form, the $pK_a$ being the negative logarithm of the add Ionization constant. The $pK_a$ will be influenced by such factors as solvent, temperature, water content, and ionic strength of the media or matrix in which the acid resides. Unless otherwise noted, the $pK_a$ Is assumed to be measured In distilled water at 25°C. Since in general, the more acidic the polymer the more useful the invention, the invention is preferred for polymers with functional groups with $pK_a$s of less than about 7, and even more preferred with $pK_a$s of less than about 6. Exemplary classes of functional groups that are included In the above description include carboxylic acids, thiocarboxylic acids, phosphates, phenolic groups, and sulfonates. Such functional groups may make up the primary structure of the polymer such as for polyacrylic acid, but more generally are covalently attached to the backbone of the parent polymer and thus are termed "substituents."

By "neutralized acidic polymer" is meant any acidic polymer for which 90% of the "acidic moieties" or "acidic substituents" have been "neutralized"; that is, exist in their deprotonated form. The "degree of neutralization," $\alpha$, of a polymer substituted with monoprotic acids (such as carboxylic acids) is defined as the fraction of the acidic moieties on the polymer that have been neutralized; that is, deprotonated by a base. The degree to which the acidic moleties on the polymer are neutralized by the base is dependent on (1) the ratio of the number of milliequivalents of base per gram of polymer divided by the number of milliequivalents of acidic moieties per gram of polymer and (2) the relative $pK_a$s of the base and the acidic polymer. When the $pK_a$ of the base Is much higher than the $pK_a$ of the acidic moieties of the addic polymer (that is, the ratio of the $pK_a$ of the base to the $pK_a$ of the polymer), then each milliequivalent of base will approximately neutralize one milliequivalent of acid. Thus, if 0.5 milliequivalent of a strong base per gram of polymer is added to an acidic polymer with 1.0 milliequivalents of acidic moieties per gram of polymer, then the degree of neutralization Is roughly equal to 0.5.

If a relatively weak base with a $pK_a$ value roughly equal to that of the polymer's acidic moieties is used to neutralize the polymer (*e.g.*, the base is the sodium salt of an aliphatic carboxylic add, such as sodium proplonate, and the acidic groups on the polymer are aliphatic carboxylic acids, such as succinate), then more base must be added to achieve the same extent of neutralization. Thus, if 1.0 milliequivalent of a base per gram of polymer, with a $pK_a$ roughly equal to the $pK_a$ of the polymer, is added to an acidic polymer with 1.0 milliequivalents of acidic moieties per gram of polymer, then the degree of neutralization is roughly also equal to 0.5.

When the degree of neutralization, $\alpha$, is less than 0.9, it may be approximated by the following equation:

$$\alpha = \frac{E_{base}}{E_{polymer}} \bullet \frac{10^{pKa,Base-pKa,Polymer}}{1+10^{pKa,Base-pKa,Polymer}}$$

where $E_{base}$ is the number of milliequivalents of base per gram of polymer, $E_{polymer}$ is the number of milliequivalents of acidic moieties (of the polymer) per gram of polymer, and $pK_a$,Base and $pK_a$,Polymer are the $pK_a$ values of the base and polymer, respectively. It should be noted that if the calculated value of $\alpha$ from this equation is greater than 1, the degree of neutralization can be considered essentially 1, meaning that essentially all of the acidic moieties on the polymer have been neutralized.

Alternatively, the degree of neutralization may be measured experimentally. Although not strictly applicable to organic solutions or solid dispersions, the Henderson-Hasselbach equation can be used to relate the effective pH of an aqueous solution or a hydrated suspension to the degree of neutralization. According to this equation the effective pH of the solution or hydrated suspension is given as:

$$pH = pK_a,Polymer - \log [(1-\alpha)/\alpha]$$

As yet another alternative, the degree of neutralization may be determined experimentally through spectroscopic analysis or thermal methods such as differential scanning calorimetry (DSC). Using DSC, for example, conversion of an acidic cellulosic polymer such as HPMCAS to the sodium or calcium salt form will lead to a measurable increase in the glass transition temperature ("$T_g$") of the polymer alone or drug/polymer dispersion. The change in physical characteristic such as glass transition temperature may be used to determine the degree of neutralization.

Often the most chemically stable compositions are formed when approximately 100% of the acidic groups of the polymer have been neutralized, that is, $\alpha$ is approximately equal to 1.0. In some cases stable dispersions are formed when excess base is present.

Yet another alternative method for determining whether a significant fraction of the acidic moieties has been neutralized is to compare the chemical stability of an HMG-CoA reductase inhibitor in a test composition comprising the HMG-CoA reductase inhibitor and a solid amorphous dispersion of a CETP inhibitor and a neutralized acidic polymer with the chemical stability of the HMG-CoA reductase Inhibitor in a control composition identical to the test composition except that the solid amorphous dispersion consists essentially of the CETP inhibitor and the acidic polymer in unneutralized form. A significant fraction of the acidic moieties of the acidic polymer have been neutralized if the HMG-CoA reductase inhibitor degrades more slowly when mixed with a solid amorphous dispersion comprising the neutralized acidic polymer relative to the rate the HMG-CoA reductase inhibitor degrades when mixed with a solid amorphous dispersion comprising the acidic polymer in unneutralized form. Thus, only a portion of the acidic moieties or acidic substituents of the polymer may need to be neutralized. Since the effective pH of an acidic polymer is raised significantly by even a small degree of neutralization, a relatively low degree of neutralization may well result in measurable improvements in the stability of the HMG-CoA reductase Inhibitor.

Neutralized acidic polymers may be either cellulosic or non-cellulosic as described above. A preferred class of acidic polymers consists of cellulosic polymers with at least one ester- and/or ether- linked acidic substituent in which the polymer has a degree of substitution of at least 0.02 for the acidic substituent. Generally, the degree of substitution of each substituent group can range from 0.02 to 2.9 as long as the other criteria of the polymer are met. More typically, the degree of substitution for each substituent is from about 0.1 to 2.0.

Exemplary acidic, ether-linked ionizable substituents include: carboxylic acids, such as carboxymethoxy (commonly referred to as carboxymethyl), carboxyethoxy (commonly referred to as carboxyethyl), carboxypropoxy (commonly referred to as carboxypropyl), and carboxyphenoxy (commonly referred to as carboxyphenyl), salicylic acid (attached to the cellulosic polymer via the phenolic hydroxyl), alkoxybenzoic acids such as ethoxybenzoic acid or propoxybenzoic acid, the various isomers of alkoxyphthalic acid such as ethoxyphthalic acid and ethoxyisophthalic acid, the various isomers of alkoxynicotinic acid such as ethoxynicotinic acid, and the various isomers of picolinic acid such as ethoxypicolinic acid, etc.; thiocarboxylic acids, such as thioacetic acid; substituted phenoxy groups, such as hydroxyphenoxy, etc.; phosphates, such as ethoxy phosphate; and sulfonates, such as ethoxy sulphonate.

Exemplary ester-linked ionizable substituents include: carboxylic acids, such as succinate, citrate, phthalate, terephthalate, isophthalate, trimellitate, and the various isomers of pyridinedicarboxylic acid, etc.; thiocarboxylic acids, such as thiosuccinate; substituted phenoxy groups, such as amino salicylic acid; phosphates, such as acetyl phosphate; and sulfonates, such as acetyl sulfonate. For aromatic-substituted polymers to also have the requisite aqueous solubility, it is also desirable that sufficient hydrophilic groups such as hydroxypropyl or carboxylic acid functional groups be attached to the polymer to render the polymer aqueous soluble at least at pH values where any ionizable groups are ionized. In some cases, the aromatic group may itself be ionizable, such as phthalate or trimellitate substituents.

Exemplary acidic cellulosic polymers include such polymers as carboxyethyl cellulose, carboxymethyl cellulose, carboxymethyl ethyl cellulose, cellulose succinate, cellulose acetate succinate, hydroxyethyl cellulose succinate, hydroxyethyl cellulose acetate succinate, hydroxyethyl methyl cellulose succinate, hydroxyethyl methyl cellulose acetate succinate, hydroxypropyl cellulose succinate, hydroxypropyl cellulose acetate succinate, hydroxypropyl methyl cellulose acetate succinate, hydroxypropyl methyl cellulose succinate, cellulose phthalate, cellulose acetate phthalate, methyl cellulose acetate phthalate, ethyl cellulose acetate phthalate, cellulose propionate phthalate, hydroxyethyl methyl cellulose acetate phthalate, hydroxypropyl cellulose acetate phthalate, hydroxypropyl methyl cellulose phthalate, hydroxypropyl methyl cellulose acetate phthalate, hydroxypropyl cellulose acetate phthalate succinate, hydroxypropyl cellulose butyrate phthalate, cellulose acetate trimellitate, methyl cellulose acetate trimellitate, ethyl cellulose acetate trimellitate, hydroxypropyl cellulose acetate trimellitate, hydroxypropyl methyl cellulose acetate trimellitate, hydroxypropyl cellulose acetate trimellitate succinate, cellulose propionate trimellitate, cellulose butyrate trimellitate, cellulose acetate terephthalate, cellulose acetate isophthalate, cellulose acetate pyridinedicarboxylate, salicylic acid cellulose acetate, hydroxypropyl salicylic acid cellulose acetate, ethylbenzoic acid cellulose acetate, hydroxypropyl ethylbenzoic acid cellulose acetate, ethyl phthalic acid cellulose acetate, ethyl nicotinic acid cellulose acetate, and ethyl picolinic acid cellulose

acetate.

Alternatively, the acidic polymer may be non-cellulosic. Exemplary acidic non-cellulosic polymers include carboxylic acid-functionalized vinyl polymers, such as the carboxylic acid functionalized polymethacrylates and carboxylic acid functionalized polyacrylates such as the EUDRAGITS® manufactured by Rohm Tech, Inc., of Malden, Massachusetts; and carboxylic acid functionalized starches such as starch glycolate.

The neutralized form of these acidic polymers often provide several advantages relative to the unneutralized form. The neutralized form of the acidic polymer, *i.e.*, the salt form of the polymer, tends to have a higher glass transition temperature relative to the acidic form of the polymer. To obtain the best physical stability, particularly upon storage for long times prior to use, it is preferred that the CETP inhibitor remain, to the extent possible, in the amorphous state. The inventors have found that this is best achieved when the mobility of the CETP inhibitor in the concentration-enhancing polymer is relatively low. This is generally the case when the glass-transition temperature, $T_g$, of the solid amorphous dispersion is substantially above the storage temperature of the dispersion. In particular, it is preferable that the $T_g$ of the solid amorphous dispersion be at least 40°C and preferably at least 60°C. It is preferred that the concentration-enhancing polymer have a $T_g$ of at least 40°C, preferably at least 70°C and more preferably greater than 100°C. (Unless otherwise specified, as used herein and in the claims, reference to a glass transition refers to the glass transition temperature measured at 50% relative humidity.) Exemplary high $T_g$ polymers include neutralized forms of hydroxypropyl methyl cellulose acetate succinate, hydroxypropyl methyl cellulose phthalate, cellulose acetate phthalate, cellulose acetate trimellitate, carboxy methyl ethyl cellulose, and other cellulosics that have alkylate or aromatic substituents or both alkylate and aromatic substituents.

Increasing the glass transition temperature of the polymer, and hence of the solid amorphous dispersion, improves the physical storage stability of the solid amorphous dispersion by decreasing the mobility of the CETP inhibitor in the polymer matrix. Thus, solid amorphous dispersions formed from neutralized acidic polymers, which have a higher $T_g$ relative to the unneutralized form, tend to be more physically stable.

When the neutralized form of the acidic polymer comprises a multivalent cationic species such as $Ca^{2+}$, $Mg^{2+}$, $Al^{3+}$, $Fe^{2+}$, $Fe^{3+}$, or a diamine, such as ethylene diamine, the cationic species may interact with two or more neutralized acidic moieties on more than one polymer chain, resulting in an ionic crosslink between the polymer chains. An acidic polymer may be considered "ionically crosslinked" if the number of milliequivalents of multivalent cationic species per gram of polymer is at least 5%, preferably at least 10% the number of milliequivalents of acidic moieties (of the polymer) per gram of polymer. Alternatively, an acidic polymer may be considered "ionically crosslinked" if sufficient multivalent cationic species are present such that the neutralized acidic polymer has a higher $T_g$ than the same polymer containing essentially no multivalent cationic species. CETP inhibitor mobility in dispersions formed from such ionically crosslinked polymers is particularly low relative to dispersions formed from the acidic form of the same polymers. Such ionically crosslinked polymers may be formed by neutralization of the acidic polymer using any base where the cationic counterion of the base is divalent. Thus, calcium hydroxide, calcium carbonate, magnesium acetate or ethylene diamine may be added to an acidic polymer such as cellulose acetate phthalate or hydroxypropyl methyl cellulose acetate succinate (HPMCAS) to form a neutralized, ionically crosslinked, acidic cellulosic polymer. Low CETP inhibitor mobility in such polymers may be indicated by high $T_g$ values or, more typically, a decrease in the magnitude of the heat capacity increase in the vicinity of the $T_g$ or, in some cases, the absence of any apparent $T_g$ when the solid amorphous dispersion is subjected to differential thermal analysis. Thus, when sufficient calcium hydroxide is added to an acidic polymer, e.g., HPMCAS, such that the degree of neutralization is near 1, no $T_g$ is apparent when the neutralized polymer is subjected to differential thermal analysis.

The neutralized form of the acidic polymer tends to be less reactive than the acidic polymer. Thus, in addition to minimizing reactions of the HMG-CoA reductase inhibitor with the polymer, the selection of a neutralized acidic enteric polymer may also minimize reactions of the polymer with other excipients.

Neutralized acidic polymers may be formed by any conventional method known in the art which results in the desired degree of neutralization. In general, the acidic polymer is neutralized through the addition of a sufficient amount of base to a solution or composition containing the acidic polymer. The polymer may be neutralized prior to formation of the solid amorphous dispersion. For example, a base may be added to a solution of the acidic polymer resulting in neutralization of the polymer's acidic functional groups. Alternatively, the acidic polymer may be neutralized during formation of the solid amorphous dispersion, or may be neutralized following formation of the solid amorphous dispersion.

A wide range of bases may be used to neutralize the acidic polymer. The term "base" is used broadly to include not only strong bases such as sodium hydroxide, but also weak bases and buffers that are capable of achieving the desired degree of neutralization. Examples of bases include hydroxides, such as sodium hydroxide, calcium hydroxide, ammonium hydroxide, and choline hydroxide; bicarbonates, such as sodium bicarbonate, potassium bicarbonate, and ammonium bicarbonate; carbonates, such as ammonium carbonate, calcium carbonate, and sodium carbonate; amines, such as tris(hydroxymethyl)amino methane, ethanolamine, diethanolamine, N-methyl glucamine, glucosamine, ethylenediamine, N,N'-dibenzylethylenediamine, N-benzyl-2-phenethylamine, cyclohexylamine, cyclopentylamine, diethylamine, isopropylamine, diisopropylamine, dodecylamine, and triethylamine; proteins, such as gelatin; amino acids such

as lysine, arginine; guanine, glycine, and adenine; polymeric amines, such as polyamino methacrylates, such as Eudragit E; conjugate bases of various acids, such as sodium acetate, sodium benzoate, ammonium acetate, disodium phosphate, trisodium phosphate, calcium hydrogen phosphate, sodium phenolate, sodium sulfate, ammonium chloride, and ammonium sulfate; salts of EDTA, such as tetra sodium EDTA; and salts of various acidic polymers such as sodium starch glycolate, sodium carboxymethyl cellulose and sodium polyacrylic acid. The use of the bicarbonates is in some cases preferred, as these generate carbon dioxide during the neutralization process, which can be removed easily following neutralization.

As described previously, dispersions that contain significant quantities of a divalent cationic or multivalent cationic species such as $Ca^{2+}$, $Mg^{2+}$, or a diamine such as ethylene diamine are particularly desirable as they may ionically crosslink the concentration-enhancing polymer. This may conveniently be accomplished by adding such species in their basic form. Thus, exemplary bases containing a dicationic species include: calcium hydroxide, calcium acetate, calcium carbonate, magnesium hydroxide, magnesium stearate, aluminum hydroxide, ethylene diamine, polyamino methyacrylate, or any other pharmaceutically acceptable compound that may form a dicationic or polycationic species in the solid amorphous dispersion.

In one neutralization method, the polymer is neutralized prior to formation of the solid amorphous dispersion. The acidic polymer is first dissolved in a suitable solvent prior to addition of the base. Suitable solvents include water; ketones, such as acetone; alcohols, such as methanol, ethanol, isopropanol; and other solvents such as tetrahydrofuran, benzene, and dichloromethane. Mixtures of solvents, including mixtures of water and one or more organic solvents, may also be used. In particular, when organic solvents are used, addition of at least a small amount of water is often preferred to facilitate the neutralization process and to minimize excessively high or low pH values. The solvent may be selected such that it is a solvent for the neutralized acidic polymer but not necessarily a solvent for the acidic polymer prior to neutralization. This may facilitate isolation of the neutralized acidic polymer. Thus, prior to adding the base, the acidic polymer is not completely dissolved in the solvent. As the base is added, the neutralized acidic polymer dissolves.

For example, the acidic polymer HPMCAS may be neutralized by addition of a base to an aqueous solution containing HPMCAS. HPMCAS has a $pK_a$ of about 5. One procedure for neutralizing HPMCAS is to suspend the HPMCAS in distilled water. A base, such as sodium bicarbonate can then be added to this solution. As the base is added, the succinate groups on HPMCAS are neutralized, forming the sodium salt form of HPMCAS and at the same time the pH of the solution increases. When the pH of the solution reaches about 5, the $pK_a$ of the acidic moieties (succinate groups) of the polymer, the degree of neutralization, $\alpha$, is 0.5. More base may be added, increasing the pH of the solution and increasing the extent of neutralization. Care must be taken, however, not to increase the pH too high, as at high pH (greater than about 8), the excess base can lead to degradation of the polymer. In the case of HPMCAS, such degradation can take the form of hydrolysis of ester-linked groups such as acetate or succinate or even cleavage of the cellulosic backbone of the polymer.

Following neutralization, the neutralized acidic polymer may be isolated and purified using methods known in the art. Examples of suitable methods include precipitation using a non-solvent, evaporation, rotoevaporation, spray-drying, and lyophilization. The neutralized acidic polymer can then be used to form the solid amorphous dispersion with the CETP inhibitor using the methods described below.

In another method, the neutralized acidic polymer is not isolated from the solvent, but instead, the CETP inhibitor is added to the polymer/solvent solution and the solid amorphous dispersion formed directly from this mixture. Examples of processes for forming the solid amorphous dispersion from such a solution are described below in connection with the discussion regarding formation of dispersions.

Another method for neutralizing an acidic concentration-enhancing polymer is to neutralize the polymer after the solid amorphous dispersion has been formed. In this method, a base is blended with the solid amorphous dispersion of CETP inhibitor and acidic polymer. Exemplary bases that may be used to neutralize the acidic polymer include any of those listed above for neutralization of a polymer in solution but include, in particular, salts of acidic polymers such as sodium starch glycolate, cross carmellose sodium, and sodium carboxymethyl cellulose; amine functionalized polymers such as aminomethacryrates, amino acrylates, chitin, and proteins; inorganic bases such as tribasic calcium phosphate, calcium carbonate, disodium hydrogen phosphate and aluminum hydroxide; salts of acidic compounds such as magnesium stearate, sodium acetate, and potassium lactate; and amines such as meglumine and mono-, di- and triethanolamine. Many of these bases, such as phosphate, carbonate and carboxylate salts, may be added in excess and as such may act as buffers, maintaining a relatively neutral pH (e.g., pH between about 5 and 9) in the solid amorphous dispersion. The amount of base to be blended with the solid amorphous dispersion should generally be in the range from about 0.1 to about 2.0 equivalents of base per equivalent of the acidic polymer moieties. In some cases, it may be necessary to add water, such as by wet granulation or by storing at elevated humidity, to speed the neutralization process.

The amount of base to be blended with the solid amorphous dispersion may be determined by various techniques. For example, the polymer and CETP inhibitor may be dissolved or slurried in water and the pH monitored as base is added. The amount of base per amount of CETP inhibitor and polymer to achieve the desired pH may be noted. Generally, adding sufficient base to substantially increase the pH may be sufficient. The amount of base required to

raise the pH to a value near 6 to 8 is often preferred.

The base and solid amorphous dispersion may be blended together to create a physical mixture using any conventional method known in the art. Thus, the base and solid amorphous dispersion may be blended together using wet- or dry-granulation. A high degree of blending or mixing is generally preferred in order to achieve maximum neutralization of the acidic polymer using this method. In general, the neutralization is facilitated by the presence of solvent, particularly water. For example, simple storage of the blended composition as a bulk material or in the form of a dosage form such as a tablet, granule or capsule under humid conditions for a period of a few hours to 30 days can result In sufficient neutralization of the acidic polymer dispersion. Likewise, the neutralization process may be facilitated by wet granulation processes in which the blend Is relatively wet during at least a portion of the processing time.

In one embodiment, the solid amorphous dispersion of CETP Inhibitor and acidic polymer is blended with calcium carbonate to partially neutralize the acidic polymer. Preferably, the weight ratio of calcium carbonate to acidic polymer is at least 0.10, more preferably at least 0.15, and most preferably at least 0.20.

Neutralization may be quantified by numerous methods, including storage and measurement of reduced drug degradation rates, spectroscopic analysis, potentiometric analysis, and thermal methods such as differential scanning calorimetry (DSC). Using DSC, for example, conversion of an acidic cellulosic polymer such as HPMCAS to the sodium or calcium salt form will lead to a measurable increase In the glass transition temperature of the polymer alone or the solid amorphous dispersion. In the case of adding calcium the glass transition may be completely absent from the DSC data.

In addition, when solid dispersions are made by thermal processes such as a melt-congeal process, or an extrusion process, using, for example, a twln-screw extruder, that may form a solid amorphous dispersion by a combination of thermal and mechanical means, then the basic excipient may be blended with the CETP Inhibitor and acidic polymer and the blend then fed to the melt-congeal or extrusion process apparatus. Such processes may also optionally include small amounts of solvent. Neutralization may occur completely or in part during processing as the heat, mechanical shear and solvent, If present, facilitate the neutralization process.

CHEMICAL STABILITY

Dosage forms in which the concentration-enhancing polymer used to form the solid amorphous dispersion is neutralized exhibit acceptably low rates of degradation of the HMG-CoA reductase inhibitor in the dosage form. The compositions and dosage forms of the present invention provide improved chemical stability of the HMG-CoA reductase inhibitor relative to a control composition. Where the composition comprises a solid amorphous dispersion of a CETP inhibitor and a neutralized acidic concentration-enhancing polymer and an HMG-CoA reductase inhibitor, the control composition is essentially the same as the composition except that the solid amorphous dispersion contains the unneutralized acidic concentration-enhancing polymer. The HPMCAS used in the control composition should have a minimum degree of substitution of sucdnate groups ($O(CO)CH_2CH_2(CO)OH$) of at least 4 wt% (or at least about 100 milliequivalents of carboxylic acid functional groups per mole of polymer). A suitable grade of HPMCAS to use in the control composition is the "H" grade, available from Shin Etsu (Tokyo, Japan).

In general, degradation of the HMG-CoA reductase inhibitor may be measured using any conventional method for measuring the potency or purity of drug In a pharmaceutical composition. For example, the amount of active HMG-CoA reductase inhibitor present In a composition may be initially measured using high-performance liquid chromatography (HPLC) or other analytical techniques well known In the art. Alternatively, the amount of HMG-CoA reductase inhibitor initially present may be calculated from the amount of drug present in the composition. The potency of the composition is then measured after storage at controlled temperature and humidity conditions for an appropriate period of time. A decrease In potency indicates that a chemical reaction has occurred, leading to a decrease in the amount of active drug present in the composition, and Is an indication of poor chemical stability.

An alternative method used to evaluate chemical stability Is to analyze the rate of increase in the amount of drug degradant(s) in the composition, which would indicate reaction of the HMG-CoA reductase inhibitor. An HPLC or other analytical technique may be used to determine the concentration of drug degradant(s) in a composition. The amount of the degradant(s) is measured before and after storage under controlled storage conditions. The amount of Increase In the drug degradant(s) may be used to determine the amount of decrease In "percent drug purity," defined as 100 times the total amount of drug present divided by the amount of drug Initially present. Thus, percent drug purity may be calculated as follows:

$$\textit{percent drug purity} = 100 \times \left( \frac{\textit{total drug present}}{\textit{drug initially present}} \right)$$

When the drug purity is calculated from the total amount of impurities, percent drug purity may be calculated by assuming that the drug initially present, given in wt%, is equal to 100 wt% minus the wt% of total initial impurities, and that total drug present is equal to 100 wt% minus the wt% of total impurities after storage, that is, at some later time. This method of calculating percent drug purity is by the formula:

$$\textit{percent drug purity} = 100 \times \left[1 - \left(\frac{\textit{total impurities}}{\textit{drug initially present}}\right)\right]$$

The rate at which drug degradation occurs is generally dependent on the storage conditions. The HMG-CoA reductase inhibitor, when formulated in a composition of the present invention, should be stable at ambient temperature and humidity conditions (*e.g.*, 20% to 60% relative humidity (RH)) for long periods of time, such as months or years. However, to expedite testing, the storage conditions may employ elevated temperature and/or humidity to simulate longer storage times at ambient conditions. The storage time may vary from a few days to weeks or months, depending on the reactivity of the drug and the storage conditions.

A "degree of degradation" of drug following storage may be determined by subtracting the final percent drug purity (determined either by measuring the decrease in drug present or the increase in drug impurities present) from the initial percent drug purity. For example, a sample of composition initially containing 100 mg HMG-CoA reductase inhibitor and having no measurable impurities would have an initial percent drug purity of 100 wt%. If, after storage, the amount of HMG-CoA reductase inhibitor in the sample decreases to 95 mg, the final percent drug purity would be 95 wt% and the degree of degradation would be 100 wt% less 95 wt%, or 5 wt%. Alternatively, if 100 mg of HMG-CoA reductase inhibitor were found to initially have 1 mg of impurities present, it would have an initial percent drug purity of 99 wt%. If, after storage, the total impurities present had increased to 6 wt%, the final percent drug purity would be 94 wt% and the degree of degradation would be 99 wt% less 94 wt%, or 5 wt%.

Alternatively, degree of degradation can be determined by subtracting the amount of one or more specific drug degradants initially present from the amount of that specific degradant present after storage. Such a measure is useful where there are several drug degradants, of which only one or a few is of concern. For example, if an HMG-CoA reductase Inhibitor Initially contained a specific degradant at a concentration of 1 wt% and after storage the concentration of that degradant was 6 wt%, the degree of degradation would be 6 wt% less 1 wt%, or 5 wt%.

A relative degree of improvement in chemical stability of the HMG-CoA reductase inhibitor In a test composition may be determined by taking the ratio of the degree of degradation of the HMG-CoA reductase inhibitor In a control composition and the degree of degradation of the HMG-CoA reductase inhibitor In a test composition under the same storage conditions for the same storage time period. The test composition is simply the composition of the solid amorphous dispersion of the CETP inhibitor and neutralized concentration-enhancing polymer, the HMG-CoA reductase inhibitor, and optional additional excipients. Where the concentation-enhancing polymer is a neutralized acidic polymer, the control composition is the same as the test composition, except that the polymer is the unneutralized form of the acidic polymer. For example, where the degree of degradation of the HMG-CoA reductase inhibitor In a test composition is 1 wt%, and the degree of degradation of the HMG-CoA reductase inhibitor In a control composition Is 5 wt%, the relative degree of improvement is 5 wt%/1 wt% equals 5.0. For compositions and dosage forms In which the solid amorphous dispersion comprises a neutralized acidic polymer, the relative degree of improvement is at least 1.1. Preferably, the relative degree of improvement is at least 1.25, more preferably at least 2.0, and even more preferably at least 3.0, most preferably at least 5.0. In fact, some compositions of the present invention may achieve a relative degree of improvement greater than 20.

The particular storage conditions and time of storage may be chosen as convenient depending on the degree of acid-sensitivity of the HMG-CoA reductase inhibitor, the particular concentration-enhancing polymer used in the solid amorphous dispersion, and the ratio of HMG-CoA reductase inhibitor to polymer in the composition. Where the HMG-CoA reductase inhibitor is particularly acid-sensitive, or where the composition has a low ratio of HMG-CoA reductase inhibitor to polymer, then shorter storage time periods may be used. Where the rate of degradation is linear, the relative degree of improvement will be Independent of the storage time. However, where the rate of degradation is non-linear under controlled storage conditions, the stability test used to compare the test composition with the control composition is preferably chosen such that the degree of degradation is sufficiently large that it may be accurately measured. Typically, the time period is chosen so as to observe a degree of degradation in the control composition of at least 0.1 wt% to 0.2 wt%. However, the time period is not so long that the ratio of HMG-CoA reductase inhibitor to polymer changes substantially. Typically, the time period Is such that the observed degree of degradation for the test composition is less than 50 wt% and preferably less than 20 wt%. When rate of degradation in the control composition is relatively slow, the test

is preferably conducted over a long enough period of time under controlled storage conditions to allow a meaningful comparison of the stability of the test composition with the control composition.

A stability test which may be used to test whether a composition or dosage form meets the chemical stability criteria described above is storage of the test dispersion and the control dispersion for six months at 40°C and 75% relative humidity (RH) or for three months at 50°C and 75% RH. A relative degree of improvement may become apparent within a shorter time, such as three to five days, and shorter storage times may be used for some very acid-sensitive HMG-CoA reductase inhibitors. When comparing dispersions under storage conditions that approximate ambient conditions, e.g., 30°C and 60% RH, the storage period may need to be several months up to two years.

In addition, it is preferred that the compositions comprising an HMG-CoA reductase inhibitor and a solid amorphous dispersion result in drug stability such that the HMG-CoA reductase inhibitor has a degree of degradation of less than about 5 wt%, more preferably less than about 2 wt%, even more preferably less than about 0.5 wt%, and most preferably less than about 0.1 wt% when stored at 40°C and 75% RH for six months, or less than about 5 wt%, more preferably less than about 2 wt%, even more preferably less than about 0.5 wt%, and more preferably less than about 0.1 wt%, when stored at 30°C and 60% RH for one year. Nevertheless, the compositions of the present invention may have a degree of degradation that is much greater than the preferred values, so long as the composition achieves the degree of improvement relative to a control composition as described above.

CHOLESTERYL ESTER TRANSFER PROTEIN INHIBITORS

A CETP inhibitor is a compound capable of inhibiting the cholesteryl ester transfer protein. Solid amorphous dispersions are particularly useful for CETP inhibitors that have sufficiently low aqueous solubility, low bioavallability or slow rate of absorption such that Is desirable to increase their concentration In an aqueous environment of use. A CETP inhibitor is typically "sparingly water-soluble," which means that the CETP inhibitor has a minimum aqueous solubility of less than about 1 to 2 mg/mL at any physiologically relevant pH (*e.g.*, pH 1-8) and at about 22°C. Many CETP inhibitors are "substantially water-Insoluble," which means that the CETP inhibitor has a minimum aqueous solubility of less than about 0.01 mg/mL (or 10 μg/ml) at any physiologically relevant pH (*e.g.*, pH 1-8) and at about 22°C. (Unless otherwise specified, reference to aqueous solubility herein and in the claims is determined at about 22°C.) Many CETP inhibitors have low solubilities (some even less than 0.1 μg/mL), and require dramatic concentration enhancement to be sufficiently bloavallable upon oral dosing for effective plasma concentrations to be reached at practical doses.

In general, CETP inhibitors have a dose-to-aqueous solubility ratio greater than about 100 mL, where the solubility (mg/mL) is the minimum value observed in any physiologically relevant aqueous solution (*e.g.*, those with pH values from 1 to 8) including USP simulated gastric and intestinal buffers, and dose is in mg. Compositions of the present invention, as mentioned above, find greater utility as the solubility of the CETP Inhibitor decreases and the dose increases. Thus, the compositions are preferred as the dose-to-solubility ratio increases, and thus are preferred for dose-to-solubility ratios greater than 1000 mL, and more preferred for dose-to-solubility ratios greater than about 5000 ml. The dose-to-solubillty ratio may be determined by dividing the dose (in mg) by the aqueous solubility (in mg/ml).

Oral delivery of many CETP inhibitors Is particularly difficult because their aqueous solubility is usually extremely low, typically being less than 2 μg/ml, often being less than 0.1 μg/ml. Such low solubilities are a direct consequence of the particular structural characteristics of species that bind to CETP and thus act as CETP inhibitors. This low solubility is primarily due to the hydrophobic nature of CETP inhibitors. Log P, defined as the base 10 logarithm of the ratio of the drug solubility in octanol to the drug solubility in water, is a widely accepted measure of hydrophobicity. Log P may be measured experimentally or calculated using methods known in the art. Calculated Log P values are often referred to by the calculation method, such as Alog P, Clog P, and Mlog P. In general, Log P values for CETP inhibitors are greater than 4 and are often greater than 5. Thus, the hydrophobic and insoluble nature of CETP inhibitors as a class pose a particular challenge for oral delivery. Achieving therapeutic drug levels in the blood by oral dosing of practical quantities of drug generally requires a large enhancement in drug concentrations in the gastrointestinal fluid and a resulting large enhancement in bioavailability. Such enhancements in drug concentration in gastrointestinal fluid typically need to be at least about 10-fold and often at least about 50-fold or even at least about 200-fold to achieve desired blood levels. Surprisingly, the solid amorphous dispersions of the present invention have proven to have the required large enhancements in drug concentration and bioavailability.

In contrast to conventional wisdom, the relative degree of enhancement in aqueous concentration and bioavailability provided by the solid amorphous dispersions generally improves for CETP inhibitors as solubility decreases and hydrophobicity increases. In fact, the inventors have recognized a subclass of these CETP inhibitors that are essentially aqueous insoluble, highly hydrophobic, and are characterized by a set of physical properties. This subclass exhibits dramatic enhancements in aqueous concentration and bioavailability when formulated using a solid amorphous dispersion.

The first property of this subclass of essentially insoluble, hydrophobic CETP inhibitors is extremely low aqueous solubility. By extremely low aqueous solubility is meant that the minimum aqueous solubility at physiologically relevant

pH (pH of 1 to 8) is less than about 10 μg/ml and preferably less than about 1 μg/ml.

A second property is a very high dose-to-solubility ratio. Extremely low solubility often leads to poor or slow absorption of the drug from the fluid of the gastrointestinal tract, when the drug is dosed orally in a conventional manner. For extremely low solubility drugs, poor absorption generally becomes progressively more difficult as the dose (mass of drug given orally) increases. Thus, a second property of this subclass of essentially insoluble, hydrophobic CETP inhibitors is a very high dose (in mg) to solubility (in mg/ml) ratio (ml). By "very high dose-to-solubility ratio" is meant that the dose-to-solubility ratio has a value of at least 1000 ml, and preferably at least 5,000 ml, and more preferably at least 10,000 ml.

A third property of this subclass of essentially insoluble, hydrophobic CETP inhibitors is that they are extremely hydrophobic. By extremely hydrophobic is meant that the Log P value of the drug, has a value of at least 4.0, preferably a value of at least 5.0, and more preferably a value of at least 5.5.

A fourth property of this subclass of essentially insoluble CETP inhibitors is that they have a low melting point. Generally, drugs of this subclass will have a melting point of about 150°C or less, and preferably about 140°C or less.

Primarily, as a consequence of some or all of these four properties, CETP inhibitors of this subclass typically have very low absolute bioavailabilities. Specifically, the absolute bioavailability of drugs in this subclass when dosed orally in their undispersed state is less than about 10% and more often less than about 5%.

For this subclass of CETP inhibitors, the CETP inhibitor, when dispersed in the solid amorphous dispersion, should be at least substantially amorphous, and more preferably is almost completely amorphous, as described below. In addition, the solid amorphous dispersion should be substantially homogeneous. As discussed below, such dispersions may be made by mechanical processes, such as milling and extrusion; melt processes, such as fusion, melt-extrusion, and melt-congealing; and solvent processes, such as non-solvent precipitation, spray coating, and spray-drying. When prepared in this fashion, this class of essentially insoluble, hydrophobic CETP inhibitors often exhibits dramatic enhancements in aqueous concentration in the use environment and in bioavailability when dosed orally. While the degree of enhancement will depend on the particular concentration-enhancing polymer, when preferred concentration-enhancing polymers are used (as discussed below), such compositions may provide a maximum drug concentration (MDC) in an aqueous use environment that is at least about 50-fold, and preferably at least about 200-fold, the equilibrium concentration of a control composition comprising an equivalent quantity of the essentially insoluble, hydrophobic CETP inhibitor but free from the concentration-enhancing polymer. Likewise, the compositions also display in an aqueous use environment an area under the concentration versus time curve (AUC), for any period of at least 90 minutes between the time of introduction into the use environment and about 270 minutes following introduction into the use environment that is at least about 25-fold, and preferably at least about 100-fold, that of the control composition comprising an equivalent quantity of drug but free from the concentration-enhancing polymer.

In the following, by "pharmaceutically acceptable forms" thereof is meant any pharmaceutically acceptable derivative or variation, including stereoisomers, stereoisomer mixtures, enantiomers, solvates, hydrates, isomorphs, polymorphs, salt forms and prodrugs.

One class of CETP inhibitors consists of (R)-chiral halogenated 1-substituted amino-(n+i)-alkanols having the Formula XVI, as described below. The CETP inhibitor with which the present invention is concerned is drawn from this class. The class consists or compounds or formula XVI

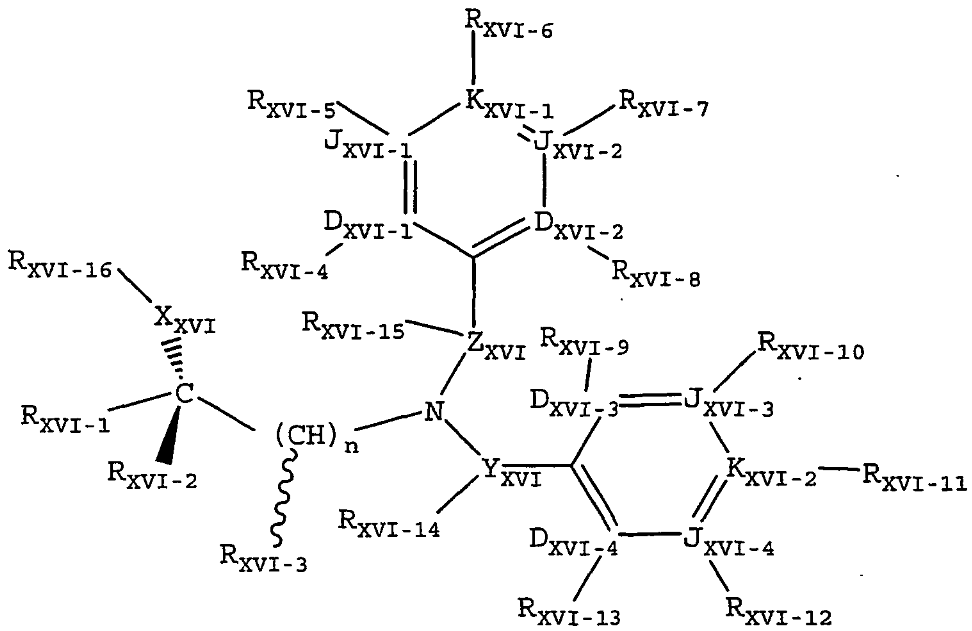

Formula XVI

and pharmaceutically acceptable forms thereof, wherein:

$n_{XVI}$ is an integer selected from 1 through 4;
$X_{XVI}$ is oxy;
$R_{XVI-1}$ is selected from the group consisting of haloalkyl, haloalkenyl, haloalkoxymethyl, and haloalkenyloxymethyl with the proviso that $R_{XVI-1}$ has a higher Cahn-Ingold-Prelog stereochemical system ranking than both $R_{XVI-2}$ and $(CHR_{XVI-3})_n$-$N(A_{XVI})Q_{XVI}$ wherein $A_{XVI}$ is Formula XVI-(II) and Q is Formula XVI-(III);

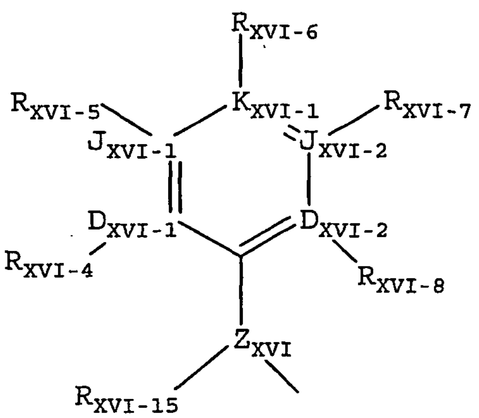

XVI-II

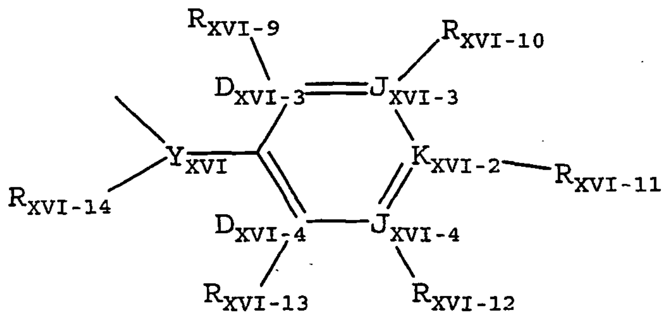

XVI-III

$R_{XVI-16}$ is selected from the group consisting of hydrido, alkyl, acyl, aroyl, heteroaroyl, trialkylsilyl, and a spacer selected from the group consisting of a covalent single bond and a linear spacer moiety having a chain length of 1 to 4 atoms linked to the point of bonding of any aromatic substituent selected from the group consisting of $R_{XVI-4}$, $R_{XVI-8}$, $R_{XVI-9}$, and $R_{XVI-13}$ to form a heterocyclyl ring having from 5 through 10 contiguous members;
$D_{XVI-1}$, $D_{XVI-2}$, $J_{XVI-1}$, $J_{XVI-2}$ and $K_{XVI-1}$ are independently selected from the group consisting of C, N, O, S and covalent bond with the provisos that no more than one of $D_{XVI-1}$, $D_{XVI-2}$, $J_{XVI-1}$, $J_{XVI-2}$ and $K_{XVI-1}$ is a covalent bond,

no more than one $D_{XVI-1}$, $D_{XVI-2}$, $J_{XVI-1}$, $J_{XVI-2}$ and $K_{XVI-1}$ is be O, no more than one of $D_{XVI-1}$, $D_{XVI-2}$, $J_{XVI-1}$, $J_{XVI-2}$ and $K_{XVI-1}$ is S, one of $D_{XVI-1}$, $D_{XVI-2}$, $J_{XVI-1}$, $J_{XVI-2}$ and $K_{XVI-1}$ must be a covalent bond when two of $D_{XVI-1}$, $D_{XVI-2}$, $J_{XVI-1}$, $J_{XVI-2}$ and $K_{XVI-1}$ are O and S, and no more than four of $D_{XVI-1}$, $D_{XVI-2}$, $J_{XVI-1}$, $J_{XVI-2}$ and $K_{XVI-1}$ is N;

$D_{XVI-3}$, $D_{XVI-4}$, $J_{XVI-3}$, $J_{XVI-4}$ and $K_{XVI-2}$ are independently selected from the group consisting of C, N, O, S and covalent bond with the provisos that no more than one is a covalent bond, no more than one of $D_{XVI-3}$, $D_{XVI-4}$, $J_{XVI-3}$, $J_{XVI-4}$ and $K_{XVI-2}$ is O, no more than one of $D_{XVI-3}$, $D_{XVI-4}$, $J_{XVI-3}$, $J_{XVI-4}$ and $K_{XVI-2}$ is S, no more than two of $D_{XVI-3}$, $D_{XVI-4}$, $J_{XVI-3}$, $J_{XVI-4}$ and $K_{XVI-2}$ is 0 and S, one of $D_{XVI-3}$, $D_{XVI-4}$, $J_{XVI-3}$, $J_{XVI-4}$ and $K_{XVI-2}$ must be a covalent bond when two of $D_{XVI-3}$, $D_{XVI-4}$, $J_{XVI-3}$, $J_{XVI-4}$ and $K_{XVI-2}$ are O and S, and no more than four of $D_{XVI-3}$, $D_{XVI-4}$, $J_{XVI-3}$, $J_{XVI-4}$ and $K_{XVI-2}$ are N;

$R_{XVI-2}$ is selected from the group consisting of hydrido, aryl, aralkyl, alkyl, alkenyl, alkenyloxyalkyl, haloalkyl, haloalkenyl, halocycloalkyl, haloalkoxy, haloalkoxyalkyl, haloalkenyloxyalkyl, halocycloalkoxy, halocycloalkoxyalkyl, perhaloaryl, perhaloaralkyl, perhaloaryloxyalkyl, heteroaryl, dicyanoalkyl, and carboalkoxycyanoalkyl, with the proviso that $R_{XVI-2}$ has a lower Cahn-Ingold-Prelog system ranking than both $R_{XVI-1}$ and $(CHR_{XVI-3})_n$-$N(A_{XVI})Q_{XVI}$;

$R_{XVI-3}$ is selected from the group consisting of hydrido, hydroxy, cyano, aryl, aralkyl, acyl, alkoxy, alkyl, alkenyl, alkoxyalkyl, heteroaryl, alkenyloxyalkyl, haloalkyl, haloalkenyl, haloalkoxy, haloalkoxyalkyl, haloalkenyloxyalkyl, monocyanoalkyl, dicyanoalkyl, carboxamide, and carboxamidoalkyl, with the provisos that $(CHR_{XVI-3})_n$-$N(A_{XVI})Q_{XVI}$ has a lower Cahn-Ingold-Prelog stereochemical system ranking than $R_{XVI-1}$ and a higher Cahn-Ingold-Prelog stereochemical system ranking than $R_{XVI-2}$;

$Y_{XVI}$ is selected from a group consisting of a covalent single bond, $(C(R_{XVI-14})_2)_q$ wherein q is an integer selected from 1 and 2 and $(CH(R_{XVI-14}))_g$-$W_{XVI}$-$(CH(R_{XVI-14}))_p$ wherein g and p are integers independently selected from 0 and 1;

$R_{XVI-14}$ is selected from the group consisting of hydrido, hydroxy, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl;

$Z_{XVI}$ is selected from a group consisting of a covalent single bond, $(C(R_{XVI-15})_2)_q$, wherein q is an integer selected from 1 and 2, and $(CH(R_{XVI-15}))_j$-$W_{XVI}$-$(CH(R_{XVI-15}))_k$ wherein j and k are integers independently selected from 0 and 1;

$W_{XVI}$ is selected from the group consisting of O, C(O), C(S),C(O)N($R_{XVI-14}$), C(S)N($R_{XVI-14}$),($R_{XVI-14}$)NC(O), ($R_{XVI-14}$ )NC(S), S, S(O), $S(C)_2$, $S(O)_2N(R_{XVI-14})$, $(R_{XVI-14})NS(O)_2$, and N($R_{XVI-14}$) with the proviso that $R_{XVI-14}$ is other than cyano;

$R_{XVI-15}$ is selected, from the group consisting of hydrido, cyano, hydroxyalkyl, acyl, alkoxy, alkyl, alkenyl, alkynyl, alkoxyalkyl, haloalkyl, haloalkenyl, haloalkoxy, haloalkoxyalkyl, haloalkenyloxyalkyl, monocarboalkoxyalkyl, monocyanoalkyl, dicyanoalkyl, carboalkoxycyanoalkyl, carboalkoxy, carboxamide, and carboxamidoalkyl;

$R_{XVI-4}$, $R_{XVI-5}$, $R_{XVI-6}$, $R_{XVI-7}$, $R_{XVI-8}$, $R_{XVI-9}$, $R_{XVI-10}$, $R_{XVI-11}$, $R_{XVI-12}$, and $R_{XVI-13}$ are independently selected from the group consisting of hydrido, carboxy, heteroaralkylthio, heteroaralkoxy, cycloalkylamino, acylalkyl, acylalkoxy, aroylalkoxy, heterocyclyloxy, aralkylaryl, aralkyl, aralkenyl, aralkynyl, heterocyclyl, perhaloaralkyl, aralkylsulfonyl, aralkylsulfonylalkyl, aralkylsulfinyl, aralkylsulfinylalkyl, halocycloalkyl, halocycloalkenyl, cycloalkylsulfinyl, cycloalkylsulfinylalkyl, cycloalkylsulfonyl, cycloalkylsulfonylalkyl, heteroarylamino, N-heteroarylamino-N-alkylamino, heteroaralkyl, heteroarylaminoalkyl, haloalkylthio, alkanoyloxy, alkoxy, alkoxyalkyl, haloalkoxylalkyl, heteroaralkoxy, cycloalkoxy, cycloalkenyloxy, cycloalkoxyalkyl, cycloalkylalkoxy, cycloalkenyloxyalkyl, cycloalkylenedioxy, halocycloalkoxy, halocycloalkoxyalkyl, halocycloalkenyloxy, halocycloalkenyloxyalkyl, hydroxy, amino, thio, nitro, lower alkylamino, alkylthio, alkylthioalkyl, arylamino, aralkylamino, arylthio, arylthioalkyl, heteroaralkoxyalkyl, alkylsulfinyl, alkylsulfinylalkyl, arylsulfinylalkyl, arylsulfonylalkyl, heteroarylsulfinylalkyl, heteroarylsulfonylalkyl, alkylsulfonyl, alkylsulfonylalkyl, haloalkylsulfinylalkyl, haloalkylsulfonylalkyl, alkylsulfonamido, alkylaminosulfonyl, amidosulfonyl, monoalkyl amidosulfonyl, dialkyl, amidosulfonyl, monoarylamidosulfonyl, arylsulfonamido, diarylamidosulfonyl, monoalkyl monoaryl amidosulfonyl, arylsulfinyl, arylsulfonyl, heteroarylthio, heteroarylsulfinyl, heteroarylsulfonyl, heterocyclylsulfonyl, heterocyclylthio, alkanoyl, alkenoyl, aroyl, heteroaroyl, aralkanoyl, heteroaralkanoyl, haloalkanoyl, alkyl, alkenyl, alkynyl, alkenyloxy, alkenyloxyalky, alkylenedioxy, haloalkylenedioxy, cycloalkyl, cycloalkylalkanoyl, cycloalkenyl, lower cycloalkylalkyl, lower cycloalkenylalkyl, halo, haloalkyl, haloalkenyl, haloalkoxy, hydroxyhaloalkyl, hydroxyaralkyl, hydroxyalkyl, hydoxyheteroaralkyl, haloalkoxyalkyl, aryl, heteroaralkynyl, aryloxy, aralkoxy, aryloxyalkyl, saturated heterocyclyl, partially saturated heterocyclyl, heteroaryl, heteroaryloxy, heteroaryloxyalkyl, arylalkenyl, heteroarylalkenyl, carboxyalkyl, carboalkoxy, alkoxycarboxamido, alkylamidocarbonylamido, arylamidocarbonylamido, carboalkoxyalkyl, carboalkoxyalkenyl, carboaralkoxy, carboxamido, carboxamidoalkyl, cyano, carbohaloalkoxy, phosphono, phosphonoalkyl, diaralkoxyphosphono, and diaralkoxyphosphonoalkyl with the proviso that $R_{XVI-4}$, $R_{XVI-5}$, $R_{XVI-6}$, $R_{XVI-7}$, $R_{XVI-8}$, $R_{XVI-9}$, $R_{XVI-10}$, $R_{XVI-11}$, $R_{XVI-12}$, and $R_{XVI-13}$ are each independently selected to maintain the tetravalent nature of carbon, trivalent nature of nitrogen, the divalent nature of sulfur, and the divalent nature of oxygen;

$R_{XVI-4}$ and $R_{XVI-5}$, $R_{XVI-5}$ and $R_{XVI-6}$, $R_{XVI-6}$, and $R_{XVI-7}$, $R_{XVI-7}$ and $R_{XVI-9}$, $R_{XVI-9}$ and $R_{XVI-10}$, $R_{XVI-10}$ and $R_{XVI-11}$,

$R_{XVI-11}$ and $R_{XVI-12}$, and $R_{XVI-12}$ and $R_{XIV-13}$ are Independently selected to form spacer pairs wherein a spacer pair is taken together to form a linear moiety having from 3 through 6 contiguous atoms connecting the points of bonding of said spacer pair members to form a ring selected from the group consisting of a cycloalkenyl ring having 5 through 8 contiguous members, a partially saturated heterocyclyl ring having 5 through 8 contiguous members, a heteroaryl ring having 5 through 6 contiguous members, and an aryl with the provisos that no more than one of the group consisting of spacer pairs $R_{XVI-4}$ and $R_{XVI-5}$, $R_{XVI-5}$ and $R_{XVI-6}$, $R_{XVI-6}$ and $R_{XVI-7}$, and $R_{XVI-7}$ and $R_{XVI-8}$ is used at the same time and that no more than one of the group consisting of spacer pairs $R_{XVI-9}$ and $R_{XVI-10}$, $R_{XVI-10}$ and $R_{XVI-11}$, $R_{XVI-11}$ and $R_{XVI-12,}$ and $R_{XVI-12}$ and $R_{XVI-13}$ can be used at the same time;

$R_{XVI-4}$ and $R_{XVI-9}$, $R_{XVI-4}$ and $R_{XVI-13}$, $R_{XVI-5}$ and $R_{XVI-9}$, and $R_{XVI-8}$ and $R_{XVI-13}$ is independently selected to form a spacer pair wherein said spacer pair Is taken together to form a linear moiety wherein said linear moiety forms a ring selected from the group consisting of a partially saturated heterocyclyl ring having from 5 through 8 contiguous members and a heteroaryl ring having from 5 through 6 contiguous members with the proviso that no more than one of the group consisting of spacer pairs $R_{XVI-4}$ and $R_{XVI-9}$, $R_{XVI-4}$ and $R_{XVI-13}$, $R_{XVI-8}$ and $R_{XVI-9}$, and $R_{XVI-8}$ and $R_{XVI-13}$ is used at the same time.

Compounds of Formula XVI are disclosed In WO 00/18724.

The following compounds are of Formula XVI:

(2R)-3-[[3-(3-trifluoromethoxyphenoxy)phenyl][[3-(1,1,2,2-tetrafluoroethoxy)phenyl]methyl]amino]-1,1,1-trifluoro-2-propanol;
(2R)-3-[[3-(3-isopropylphenoxy)phenyl][[3-(1,1,2,2-tetrafluoroethoxy)phenyl]-methyl]amino]-1,1,1-trifluoro-2-propanol;
(2R)-3-[[3-(3-cyclopropylphenoxy)phenyl][[3-(1,1,2,2-tetrafluoroethoxy)phenyl]-methyl]amino]-1,1,1-trifluoro-2-propanol;
(2R)-3-[[3-(3-(2-furyl)phenoxy)phenyl][[3-(1,1,2,2-tetrafluoroethoxy)phenyl]-methyl]amino]-1,1,1-trifluoro-2-propanol;
(2R)-3-[[3-(2,3-dichlorophenoxy)phenyl][[3-(1,1,2,2-tetrafluoroethoxy)phenyl]-methyl]amino]-1,1,1-trifluoro-2-propanol;
(2R)-3-[[3-(4-fluorophenoxy)phenyl][[3-(1,1,2,2-tetrafluoroethoxy)phenyl]-methyl]amino]-1,1,1-trifluoro-2-propanol;
(2R)-3-[[3-(4-methylphenoxy)phenyl][[3-(1,1,2,2-tetrafluoroethoxy)phenyl]-methyl]amino]-1,1,1-trifluoro-2-propanol;
(2R)-3-[[3-(2-fluoro-5-bromophenoxy)phenyl][[3-(1,1,2,2-tetrafluoroethoxy)phenyl]-methyl]amino]-1,1,1-trifluoro-2-propanol;
(2R)-3-[[3-(4-chloro-3-ethylphenoxy)phenyl][[3-(1,1,2,2-tetrafluoroethoxy)phenyl]-methyl]amino]-1,1,1-trifluoro-2-propanol;
(2R)-3-[[3-[3-(1,1,2,2-tetrafluoroethoxy)phenoxy]phenyl][[3-(1,1,2,2-tetrafluoro-ethoxy)phenyl]methyl]amino]-1,1,1-trifluoro-2-propanol;
(2R)-3-[[3-[3-(pentafluoroethyl)phenoxy]phenyl][[3-(1,1,2,2-tetrafluoroethoxy)-phenyl]methyl]amino]-1,1,1-trifluoro-2-propanol;
(2R)-3-[[3-(3,5-dimethylphenoxy)phenyl][[3-(1,1,2,2-tetrafluoroethoxy)phenyl]-methyl]amino]-1,1,1-trifluoro-2-propanol;
(2R)-3-[[3-(3-ethylphenoxy)phenyl][[3-(1,1,2,2-tetrafluoroethoxy)phenyl]-methyl]amino]-1,1,1-trifluoro-2-propanol;
(2R)-3-[[3-(3-t-butylphenoxy)phenyl][[3-(1,1,2,2-tetrafluoroethoxy)phenyl]-methyl]amino]-1,1,1-trifluoro-2-propanol:
(2R)-3-[[3-(3-methylphenoxy)phenyl][[3-(1,1,2,2-tetrafluoroethoxy)phenyl]-methyl]amino]-1,1,1-trifluoro-2-propanol;
(2R)-3-[[3-(5,6,7,8-tetrahydro-2-naphthoxy)phenyl][[3-(1,1,2,2-tetrafluoro-ethoxy)phenyl]methyl]amino]-1,1,1-trifluoro-2-propanol;
(2R)-3-[[3-(phenoxy)phenyl][[3-(1,1,2,2-tetrafluoroethoxy)phenyl]methyl]amino]-1,1,1-trifluoro-2-propanol;
(2R)-3-[[3-[3-(N,N-dimethylamino)phenoxy]phenyl][[3-(1,1,2,2-tetrafluoro-ethoxy)phenyl]methyl]amino]-1,1,1-trifluoro-2-propanol;
(2R)-3-[[[3-(1,1,2,2,-tetrafluoroethoxy)phenyl]methyl][3-[[3-(trifluoromethoxy)-phenyl]methoxy]phenyl]amino]-1,1,1-trifluoro-2-propanol;
(2R)-3-[[[3-(1,1,2,2-tetrafluoroethoxy)phenyl]methyl][3-[[3-(trifluoromethyl)phenyl]methoxy]phenyl]amino]-1,1,1-trifluoro-2-propanol;
(2R)-3-[[[3-(1,1,2,2-tetrafluoroethoxy)phenyl]methyl][3-[[3,5-dimethylphenyl]-methoxy]phenyl]amino]-1,1,1-trifluoro-2-propanol;
(2R)-3-[[[3-(1,1,2,2-tetrafluoroethoxy)phenyl]methyl][3-[[3-(trifluoromethylthio)-phenyl]methoxy]phenyl]amino]-1,1,1-trifluoro-2-propanol;
(2R)-3-[[[3-(1,1,2,2-tetrafluoroethoxy)phenyl]methyl][3-[[3,5-difluorophenyl]-methoxy]phenyl]amino]-1,1,1-trifluoro-2-propanol;
(2R)-3-[[[3-(1,1,2,2-tetrafluoroethoxy)phenyl]methyl][3-[cyclohexylmethoxy]-phenyl]amino]-1,1,1-trifluoro-2-propanol;
(2R)-3-[[3-(2-difluoromethoxy-4-pyridyloxy)phenyl][[3-(1,1,2,2-tetrafluoroethoxy)-phenyl]methyl]amino]-1,1,1-trifluoro-2-propanol;
(2R)-3-[[3-(2-trifluoromethyl-4-pyridyloxy)phenyl][[3-(1,1,2,2-tetrafluoroethoxy)-phenyl]methyl]amino]-1,1,1-trifluoro-2-propanol;
(2R)-3-[[3-(3-difluoromethoxyphenoxy)phenyl][[3-(1,1,2,2-tetrafluoroethoxy)-phenyl]methyl]amino]-1,1,1-trifluoro-2-propanol;
(2R)-3-[[[3-(3-trifuoromethylthio)phenoxy]phenyl][[3-(1,1,2,2-tetrafluoroethoxy)-phenyl]methyl]amino]-1,1,1-trifluoro-2-propanol;
(2R)-3-[[3-(4-chloro-3-trifluoromethylphenoxy)phenyl][[3-( 1,1,2,2-tetrafluoroethoxy)-phenyl]methyl]amino]-1,1,1-trifluoro-2-propanol;
(2R)-3-[[3-(3-trifluoromethoxyphenoxy)phenyl][[3-(pentafluoroethyl)phenyl]-methyl]amino]-1,1,1-trifluoro-2-propanol;
(2R)-3-[[3-(3-isopropylphenoxy)phenyl][[3-(pentafluoroethyl)phenyl]methyl]-amino]-1,1,1-trifluoro-2-propanol;
(2R)-3-[[3-(3-cyclopropylphenoxy)phenyl][[3-(pentafluoroethyl)phenyl]methyl]-amino]-1,1,1-trifluoro-2-propanol;
(2R)-3-[[3-(3-(2-furyl)phenoxy)phenyl][[3-(pentafluoroethyl)phenyl]methyl]-amino]-1,1,1-trifluoro-2-propanol;
(2R)-3-[[3-(2,3-dichlorophenoxy)phenyl][[3-(pentafluoroethyl)phenyl]methyl]-amino]-1,1,1-trifluoro-2-propanol;
(2R)-3-[[3-(4-fluorophenoxy)phenyl][[3-(pentafluoroethyl)phenyl]methyl]amino]-1,1,1-trifluoro-2-propanol;
(2R)-3-[[3-(4-methylphenoxy)phenyl][[3-(pentafluoroethyl)phenyl]methyl]amino]-1,1,1-trifluoro-2-propanol;
(2R)-3-[[3-(2-fluoro-5-bromophenoxy)phenyl][[3-(pentafluoroethyl)phenyl]methyl]-amino]-1,1,1-trifluoro-2-propanol;
(2R)-3-[[3-(4-chloro-3-ethylphenoxy)phenyl][[3-(pentafluoroethyl)phenyl]methyl]-amino]-1,1,1-trifluoro-2-propanol;
(2R)-3-[[3-[3-(1,1,2,2-tetrafluoroethoxy)phenoxy]phenyl][[3-(pentafluoroethyl)-phenyl]methyl]amino]-1,1,1-trifluoro-2-propanol;
(2R)-3-[[3-[3-(pentafluoroethyl)phenoxy]phenyl][[3-(pentafluoroethyl)phenyl]-methyl]amino]-1,1,1-trifluoro-2-propanol;
(2R)-3-[[3-(3,5-dimethylphenoxy)phenyl][[3-(pentafluoroethyl) phenyl]methyl]-amino]-1,1,1-trifluoro-2-propanol;
(2R)-3-[[3-(3-ethylphenoxy)phenyl][[3-(pentafluoroethyl) phenyl]methyl]amino]-1,1,1-trifluoro-2-propanol;
(2R)-3-[[3-(3-t-butylphenoxy)phenyl][[3-(pentafluoroethyl) phenyl]methyl]amino]-1,1,1-trifluoro-2-propanol;
(2R)-3-[[3-(3-methylphenoxy)phenyl][[3-(pentafluoroethyl) phenyl]methyl]amino]-1,1,1-trifluoro-2-propanol;
(2R)-3-[[3-(5,6,7,8-tetrahydro-2-naphthoxy)phenyl][[3-(pentafluoroethyl)phenyl]-methyl]amino]-1,1,1-trifluoro-2-propanol;
(2R)-3-[[3-(phenoxy)phenyl][[3(pentafluoroethyl)phenyl]methyl]amino]-1,1,1-trifluoro-2-propanol;
(2R)-3-[[3-[3-(N,N-dimethylamino)phenoxy]phenyl][[3-(pentafluoroethyl)phenyl]-methyl]amino]-1,1,1-trifluoro-2-propanol;
(2R)-3-[[[3-(pentafluoroethyl)phenyl]methyl][3-[[3-(trifluoromethoxy)phenyl]-methoxy]phenyl]amino]-1,1,1-trifluoro-2-propanol;
(2R)-3-[[[3-(pentafluoroethyl)phenyl]methyl][3-[[3-(trifluoromethyl)-phenyl]-methoxy]phenyl]amino]-1,1,1-trifluoro-2-propanol;
(2R)-3-[[[3-(pentafluoroethyl)phenyl]methyl][3-[[3,5-dimethylphenyl]methoxy]-phenyl]amino]-1,1,1-trifluoro-2-propanol;
(2R)-3-[[[3-(pentafluoroethyl)phenyl]methyl][3-[[3-(trifluoromethylthio)phenyl]-methoxy]phenyl]amino]-1,1,1-trifluoro-2-propanol;
(2R)-3-[[[3-(pentafluoroethyl)phenyl]methyl][3-[[3,5-difluorophenyl]methoxy]-phenyl]amino]-1,1,1-trifluoro-2-propanol;
(2R)-3-[[[3-(pentafluoroethyl)phenyl]methyl][3-[cyclohexylmethoxy]phenyl]-amino]-1,1,1-trifluoro-2-propanol;
(2R)-3-[[3-(2-difluoromethoxy-4-pyridyloxy)phenyl][[3-(pentafluoroethyl)phenyl]-methyl]amino]-1,1,1-trifluoro-2-propanol;
(2R)-3-[[3-(2-trifluoromethyl-4-pyridyloxy)phenyl][[3-(pentafluoroethyl)phenyl]-methyl]amino]-1,1,1-trifluoro-2-propanol;
(2R)-3-[[3-(3-difluoromethoxyphenoxy)phenyl][[3-(pentafluoroethyl)phenyl]-methyl]amino]-1,1,1-trifluoro-2-propanol;
(2R)-3-[[[3-(3-trifluoromethylthio)phenoxy]phenyl][[3-(pentafluoroethyl)phenyl]-methyl]amino]-1,1,1-trifluoro-2-propanol;

(2R)-3-[[3-(4-chloro-3-trifluoromethylphenoxy)phenyl][[3-(pentafluoroethyl)-phenyl]methyl]amino]-1,1,1-trifluoro-2-propanol;
(2R)-3-[[3-(3-trifluoromethoxyphenoxy)phenyl][[3-(heptafluoropropyl)phenyl]-methyl]amino]-1,1,1-trifluoro-2-propanol;
(2R)-3-[[3-(3-isopropylphenoxy)phenyl][[3-(heptafluoropropyl)phenyl]methyl]-amino]-1,1,1-trifluoro-2-propanol;
(2R)-3-[[3-(3-cyclopropylphenoxy)phenyl][[3-(heptafluoropropyl)phenyl]methyl]-amino]-1,1,1-trifluoro-2-propanol;
(2R)-3-[[3-(3-(2-furyl)phenoxy)phenyl][[3-(heptafluoropropyl) phenyl]methyl]-amino]-1,1,1-trifluoro-2-propanol;
(2R)-3-[[3-(2,3-dichlorophenoxy)phenyl][[3-(heptafluoropropyl) phenyl]methyl]-amino]-1,1,1-trifluoro-2-propanol;
(2R)-3-[[3-(4-fluorophenoxy)phenyl][[3-(heptafluoropropyl) phenyl]methyl]amino]-1,1,1-trifluoro-2-propanol;
(2R)-3-[[3-(4-methylphenoxy)phenyl][[3-(heptafluoropropyl) phenyl]methyl]amino]-1,1,1,-trifluoro-2-propanol;
(2R)-3-[[3-(2-fluoro-5-bromophenoxy)phenyl][[3-(heptafluoropropyl)phenyl]-methyl]amino]-1,1,1-trifluoro-2-propanol;
(2R)-3-[[3-(4-chloro-3-ethylphenoxy)phenyl][[3-(heptafluoropropyl)phenyl]methyl]-amino]-1,1,1-trifluoro-2-propanol;
(2R)-3-[[3-[3-(1,1,2,2-tetrafluoroethoxy)phenoxy]phenyl][ [3-(heptafluoropropyl)-phenyl]methyl]amino]-1,1,1-trifluoro-2-propanol;
(2R)-3-[[3-[3-(pentafluoroethyl)phenoxy]phenyl][[3-(heptafluoropropyl)phenyl]-methyl]amino]-1,1,1-trifluoro-2-propanol;
(2R)-3-[[3-(3,5-dimethylphenoxy)phenyl][[3-(heptafluoropropyl) phenyl]methyl]-amino]-1,1,1-trifluoro-2-propanol;
(2R)-3-[[3-(3-ethylphenoxy)phenyl][[3-(heptafluoropropyl) phenyl]methyl]amino]-1,1,1-trifluoro-2-propanol;
(2R)-3-[[3-(3-t-butylphenoxy)phenyl][[3-(heptafluoropropyl) phenyl]methyl]amino]-1,1,1-trifluoro-2-propanol;
(2R)-3-[[3-(3-methylphenoxy)phenyl][[3-(heptafluoropropyl) phenyl]methyl]amino]-1,1,1-trifluoro-2-propanol;
(2R)-3-[[3-(5,6,7,8-tetrahydro-2-naphthoxy)phenyl][[3-(heptafluoropropyl)phenyl]-methyl]amino]-1,1,1-trifluoro-2-propanol;
(2R)-3-[[3-(phenoxy)phenyl][[3-(heptafluoropropyl)phenyl]methyl]amino]-1,1,1-trifluoro-2-propanol;
(2R)-3-[[3-[3-(N,N-dimethylamino)phenoxy]phenyl][[3-(heptafluoropropyl)phenyl]-methyl]amino]-1,1,1-trifluoro-2-propanol;
(2R)-3-[[[3-(heptafluoropropyl)phenyl]methyl][3-[[3-(trifluoromethoxy)phenyl]-methoxy]phenyl]amino]-1,1,1-trifluoro-2-propanol;
(2R)-3-[[[3-(heptafluoropropyl)phenyl]methyl][3-[[3-(trifluoromethyl)phenyl]-methoxy]phenyl]amino]-1,1,1-trifluoro-2-propanol;
(2R)-3-[[[3-(heptafluoropropyl)phenyl]methyl][3-[[3,5-dimethylphenyl]methoxy]-phenyl]amino]-1,1,1-trifluoro-2-propanol;
(2R)-3-[[[3-(heptafluoropropyl)phenyl]methyl][3-[[3-(trifluoromethylthio)phenyl]-methoxy]phenyl]amino]-1,1,1-trifluoro-2-propanol;
(2R)-3-[[[3-(heptafluoropropyl)phenyl]methyl][3-[[3,5-difluorophenyl]methoxy]-phenyl]amino]-1,1,1-trifluoro-2-propanol;
(2R)-3-[[[3-(heptafluoropropyl)phenyl]methyl][3-[cyclohexylmethoxy]phenyl]-amino]-1,1,1-trifluoro-2-propanol;
(2R)-3-[[3-(2-difluoromethoxy-4-pyridyloxy)phenyl][[3-(heptafluoropropyl)phenyl]-methyl]amino]-1,1,1-trifluoro-2-propanol;
(2R)-3-[[3-(2-trifluoromethyl-4-pyridyloxy)phenyl][[3-(heptafluoropropyl)phenyl]-methyl]amino]-1,1,1-trifluoro-2-propanol;
(2R)-3-[[3-(3-difluoromethoxyphenoxy)phenyl][[3-(heptafluoropropyl)phenyl]-methyl]amino]-1,1,1-trifluoro-2-propanol;
(2R)-3-[[[3-(3-trifluoromethylthio)phenoxy]phenyl][[3-(heptafluoropropyl)phenyl]-methyl]amino]-1,1,1-trifluoro-2-propanol;
(2R)-3-[[3-(4-chloro-3-trifluoromethylphenoxy)phenyl][[3-(heptafluoropropyl)-phenyl]methyl]amino]-1,1,1-trifluoro-2-propanol;
(2R)-3-[[3-(3-trifluoromethoxyphenoxy)phenyl][[2-fluoro-5-(trifluoromethyl)-phenyl]methyl]amino]- 1,1,1 -trifluoro-2-propanol;
(2R)-3-[[3-(3-isopropylphenoxy)phenyl][[2-fluoro-5-(trifluoromethyl )phenyl]-methyl]amino]-1,1,1-trifluoro-2-propanol;
(2R)-3-[[3-(3-cyclopropylphenoxy)phenyl][[2-fluoro-5-(trifluoromethyl)phenyl]-methyl]amino]-1,1,1-trifluoro-2-propanol;
(2R)-3-[[3-(3-(2-furyl)phenoxy)phenyl][[2-fluoro-5-(trifluoromethyl)phenyl]-methyl]amino]-1,1,1-trifluoro-2-propanol;
(2R)-3-[[3-(2,3-dichlorophenoxy)phenyl][[2-fluoro-5-(trifluoromethyl)phenyl]-methyl]amino]-1,1,1-trifluoro-2-propanol;
(2R)-3-[[3-(4-fluorophenoxy)phenyl][[2-fluoro-5-(trifluoromethyl)phenyl]-methyl]amino]-1,1,1-trifluoro-3-propanol;

(2R)-3-[[3-(4-methylphenoxy)phenyl][[2-fluoro-5-(trifluoromethyl)phenyl]-methyl]amino]-1,1,1-trifluoro-2-propanol;

(2R)-3-[[3-(2-fluoro-5-bromophenoxy)phenyl][[2-fluoro-5-(trifluoromethyl)-phenyl]methyl]amino]-1,1,1-trfluoro-2-propanol;

(2R)-3[[3-(4-chloro-3-ethylphenoxy)phenyl][[2-fluoro-5-(trifluoromethyl)-phenyl]methyl]amino]-1,1,1-trifluoro-2-propanol;

(2R)-3-[[3-[3-(1,1,2,2-tetrafluoroethoxy)phenoxy]phenyl][[2-fluoro-5-(trifluoromethyl)phenyl]methyl]amino]-1,1,1-trifluoro-2-propanol;

(2R)-3-[[3-[3-(pentafluoroethyl)phenoxy]phenyl][[2-fluoro-5-(trifluoromethyl)-phenyl]methyl]amino]-1,1,1-trifluoro-2-propanol;

(2R)-3-[[3-(3,5-dimethylphenoxy)phenyl][[2-fluoro-5-(trifluoromethyl)phenyl]-methyl]amino]-1,1,1-trifluoro-2-propanol;

(2R)-3-[[3-(3-ethylphenoxy)phenyl][[2-fluoro-5-(trifluoromethyl)phenyl]methyl]-amino]-1,1,1-trifluoro-2-propanol;

(2R)-3-[[3-(3-t-butylphenoxy)phenyl][[2-fluoro-5-(trifluoromethyl)phenyl]methyl]-amino]-1,1,1-trifluoro-2-propanol;

(2R)-3-[[3-(3-methylphenoxy)phenyl][[2-fluoro-5-(trifluoromethyl)phenyl]methyl]-amino]-1,1,1-trifluoro-2-propanol;

(2R)-3-[[3-(5,6,7,8-tetrahydro-2-naphthoxy)phenyl][[2-fluoro-5-(trifluoromethyl)-phenyl]methyl]amino]-1,1,1-trifluoro-2-propanol;

(2R)-3-[[3-(phenoxy)phenyl][[2-fluoro-5-(trifluoromethyl) phenyl]methyl]amino]-1,1,1-trifluoro-2-propanol;

(2R)-3-[[3-[3-(N,N-dimethylamino,phenoxy]phenyl][[2-fluoro-5-(trifluoromethyl)-phenyl]methyl]amino]-1,1,1-trifluoro-2-propanol;

(2R)-3-[[[2-fluoro-5-(trifluoromethyl)phenyl]methyl][3-[[3-(trifluoromethoxy)-phenyljmethoxy]phenyl]amino]-1,1,1-trifluoro-3-propanol;

(2R)-3-[[[2-fluoro-5-(trifluoromethyl)phenyl]methyl][3-[[3-(trifluoromethyl)-phenyl]methoxy]phenyl]amino]-1,1,1-trifluoro-2-propanol;

(2R)-3-[[[2-fluoro-5-(trifluoromethyl)phenyl]methyl][3-[[3,5-dimethylphenyl]-methoxy]phenyl]amino]-1,1,1-trifluoro-2-propanol;

(2R)-3-[[[2-fluoro-5-(trifluoromethyl)phenyl]methyl][3-[[3-(trifluoromethylthio)-phenyl]methoxy]phenyl]amino]-1,1,1-trifluoro-2-propanol;

(2R)-3-[[[2-fluoro-5-(trifluoromethyl)phenyl]methyl][3-[[3,5-difluorophenyl]-methoxy]phenyl]amino]-1,1,1-trifluoro-2-propanol;

(2R)-3-[[[2-fluoro-5-(trifluoromethyl)phenyl]methyl][3-[cyclohexylmethoxyl-phenyl]amino]-1,1,1-trifluoro-2-propanol;

(2R)-3-[[3-(2-difluoromethoxy-4-pyridyloxy)phenyl][[2-fluoro-5-(trifluoromethyl)-phenyl]methyl]amino]-1,1,1-tifluoro-2-propanol;

(2R)-3-[[3-(2-trifluoromethyl-4-pyridyloxy)phenyl][[2-fluoro-5-(trifluoromethyl)-phenyl]methyl]amino]-1,1,1-trifluoro-2-propanol;

(2R)-3-[[3-(3-difluoromethoxyphenoxy)phenyl][[2-fluoro-5-(trifluoromethyl)-phenyl]methyl]amino]-1,1,1-trifluoro-2-propanol;

(2R)-3-[[[3-(3-trifluoromethylthio)phenoxy]phenyl][[2-fluoro-5-(trifluoromethyl)-phenyl]methyl]amino]-1,1,1-trifluoro-2-propanol;

(2R)-3-[[3-(4-chloro-3-trifluoromethylphenoxy)phenyl][[2-fluoro-5-(trifluoromethyl)phenyl]methyl]amino]-1,1,1-trifluoro-2-propanol;

(2R)-3-[[3-(3-trifluoromethoxyphenoxy)phenyl][[2-fluoro-4-(trifluoromethyl)-phenyl]methyl]amino]-1,1,1-trifluoro-2-propanol;

(2R)-3-[[3-(3-isopropylphenoxy)phenyl][[2-fluoro-4-(trifluoromethyl)phenyl]-methyl]amino]I-1,1,1-trifluoro-2-propanol;

(2R)-3-[[3-(3-cyclopropylphenoxy)phenyl][[2-flouro-4-(trifluoromethyl)phenyl]-methyl]amino]-1,1,1-trifluoro-2-propanol;

(2R)-3-[[3-(3-(2-furyl)phenoxy)phenyl][[2-fluoro-4-(trifluoromethyl)phenyl]-methyl]amino]-1,1,1-trifluoro-2-propanol;

(2R)-3-[[3-(2,3-dichlorophenoxy)phenyl][[2-fluoro-4-(trifluoromethyl)phenyl]-methyl]amino]-1,1,1-trifluoro-2-propanol;

(2R)-3-[[3-(4-fluorophenoxy)phenyl][[2-fluoro-4-(trifluoromethyl)phenyl]-methyl]amino]-1,1,1-trifluoro-2-propanol;

(2R)-3-[[3-(4-methylphenoxy)phenyl][[2-fluoro-4-(trifluoromethyl)phenyl]-methyl]amino]-1,1,1-trifluoro-2-propanol;

(2R)-3-[[3-(2-fluoro-5-bromophenoxy)phenyl][[2-fluoro-4-(trifluoromethyl)-phenyl]methyl]amino]-1,1,1-trifluoro-2-propanol;

(2R)-3-[[3-(4-chloro-3-ethylphenoxy)phenyl][[2-fluoro-4-(trifluoromethyl)-phenyl]methyl]amino]-1,1,1-trifluoro-2-propanol;

(2R)-3-[[3-[3-(1,1,2,2-tetrafluoroethoxy)phenoxy]phenyl][[2-fluoro-4-(trifluoromethyl)phenyl]methyl]amino]-1,1,1-trifluoro-2-propanol;

(2R)-3-[[3-[3-(pentafluoroethyl)phenoxy]phenyl][[2-fluoro-4-(trifluoromethyl)-phenyl]methyl]amino]-1,1,1-trifluoro-2-propanol;
(2R)-3-[[3-(3,5-dimethylphenoxy)phenyl][[2-fluoro-4-(trifluoromethyl)phenyl]-methyl]aminol-1,1,1-trifluoro-2-propanol;
(2R)-3-[[3-(3-ethylphenoxy)phenyl][[2-fluoro-4-(trifluoromethyl)phenyl]methyl]-amino]-1,1,1-trifluoro-2-propanol;
(2R)-3-[[3-(3-t-butylphenoxy)phenyl][[2-fluoro-4-(trifluoromethyl)phenyl]methyl]-amino]-1,1,1-trifluoro-2-propanol;
(2R)-3-[[3-(3-methylphenoxy)phenyl][[2-fluoro-4-(trifluoromethyl)phenyl]methyl]-amino]-1,1,1-trifluoro-2-propanol;
(2R)-3-[[3-(5,6,7,8-tetrahydro-2-naphthoxy)phenyl][[2-fluoro-4-(trifluoromethyl)-phenyl]methyl]amino]-1,1,1-trifluoro-2-propanol;
(2R)-3-[[3-(phenoxy)phenyl][[2-fluoro-4-(trifluoromethyl)phenyl]methyl]amino]-1,1,1-trifluoro-2-propanol;
(2R)-3-[[3-[3-(N,N-dimethylamino)phenoxy]phenyl][[2-fluoro-4-(trifluoromethyl)-phenyl]methyl]amino]-1,1,1-trifluoro-2-propanol;
(2R)-3-[[[2-fluoro-4-(trifluoromethyl)phenyl]methyl][3-[[3-(trifluoromethoxy)phenyl]methoxy]phenyl]amino]-1,1,1-trifluoro-2-propanol;
(3R)-3-[[[2-fluoro-4-(trifluoromethyl)phenyl]methyl][3-[[3-(trifluoromethyl)phenyl]methoxy]phenyl]amino]-1,1,1-trifluoro-2-propanol;
(2R)3-[[[2-fluoro-4-(trifluoromethyl)phenyl]methyl][3-[[3,5-dimethylphenyl]-methoxy]phenyl]amino]-1,1,1-trifluoro-2-propanol;
(2R)-3-[[[2-fluoro-4-(trifluoromethyl)phenyl]methyl][3-[[3-(trifluoromethylthio)-phenyl]methoxy]phenyl]amino]-1,1,1-trifluoro-2-propanol;
(2R)-3-[[[2-fluoro-4-(trifluoromethyl)phenyl]methyl][3-[[3,5-difluorophenyl]-methoxy]phenyl]amino]-1,1,1-trifluoro-2-propanol;
(2R)-3-[[[2-fluoro-4-(trifluoromethyl)phenyl]methyl][3-[cyclohexylmethoxy]-phenyl]amino]-1,1,1-trifluoro-2-propanol;
(2R)-3-[[3-(2-difluoromethoxy-4-pyridyloxy)phenyl][[2-fluoro-4-(trifluoromethyl)-phenyl]methyl]amino]-1,1,1-trifluoro-2-propanol;
(2R)-3-[[3-(2-trifluoromethyl-4-pyridyloxy)phenyl][[2-fluoro-4-(trifluoromethyl)-phenyl]methyl]amino]-1,1,1-trifluoro-2-propanol;
(2R)-3-[[3-(3-difluoromethoxyhenoxy)phenyl][[2-fluoro-4-(trifluoromethyl)-phenyl]methyl]amino]-1,1,1-bifluoro-2-propanol;
(2R)-3-[[[3-(3-trifluoromethylthlo)phenoxy]phenyl][[2-fluoro-4-(trifluoromethyl)-phenyl]methyl]amino]-1,1,1-trifluoro-2-propanol; and
(2R)-3-[[3-(4-chloro-3-trifluoromethylphenoxy)phenyl][[2-fluoro-4-(trifluoromethyl)phenyl]methyl]amino]-1,1,1-trifluoro-2-propanol.

## CONCENTRATION ENHANCEMENT

The polymer used in the solid amorphous dispersion is a "concentration-enhancing polymer," meaning that it meets at least one, and preferably both, of the following conditions. The first condition is that the concentration-enhancing polymer increases the maximum drug concentration (MDC) of the CETP inhibitor provided by either the solid amorphous dispersion alone, or the dosage form, in the environment of use relative to a control composition. That is, once the solid amorphous dispersion or dosage form is introduced Into an environment of use, the polymer increases the aqueous concentration in the use environment of CETP inhibitor relative to the control composition. It is to be understood that the control composition is free from solubilizers or other components that would materially affect the solubility of the CETP inhibitor, and that the CETP inhibitor is in solid form in the control composition. The control composition is conventionally the undispersed, or crystalline form, of the CETP inhibitor alone. In the case of a dosage form, the control composition is the same as the dosage form, except that the solid amorphous dispersion is replaced by undispersed CETP inhibitor and no polymer, the amount of CETP inhibitor being equivalent to the amount in the solid amorphous dispersion. Preferably, the polymer increases the MDC of the CETP inhibitor in the aqueous use environment by at least 1.25-fold relative to a control composition, more preferably by at least 2-fold, and most preferably by at least 3-fold. Surprisingly, the polymer may achieve extremely large enhancements in aqueous concentration. In some cases, the MDC of CETP inhibitor provided by the test composition is at least 10-fold, at least 50-fold, at least 200-fold, at least 500-fold, to more than 1000-fold the equilibrium concentration provided by the control composition, where the test composition is either the solid amorphous dispersion or the dosage form.

The second condition is that the concentration-enhancing polymer increases the area under the concentration versus time curve (AUC) of the CETP inhibitor in the environment of use relative to a control composition consisting of the undispersed CETP inhibitor but no polymer. (The calculation of an AUC is a well-known procedure in the pharmaceutical arts and is described, for example, in Welling, "Pharmacokinetics Processes and Mathematics," ACS Monograph

185 (1986).) More specifically, in the environment of use, the solid amorphous dispersion comprising the CETP inhibitor and the concentration-enhancing polymer provides an AUC for any 90-minute period of from about 0 to about 270 minutes following introduction to the use environment that is at least 1.25-fold that of the control composition described above. Preferably, the AUC provided by the solid amorphous dispersion is at least 2-fold, more preferably at least 3-fold that of the control composition. For some CETP inhibitors, the compositions of the present invention may provide an AUC value that is at least 5-fold, at least 25-fold, at least 100-fold, and even more than 250-fold that of a control composition as described above.

As previously mentioned, a "use environment" can be either the *in vivo* environment such as the GI tract of an animal, particularly a human, or the *in vitro* environment of a test solution, such as phosphate buffered saline (PBS) solution or Model Fasted Duodenal (MFD) solution.

Concentration enhancement may be determined through either *in vivo* tests or through *in vitro* dissolution tests. A composition of the present invention meets the concentration enhancement criteria in at least one of the above test environments.

Where the use environment is the GI tract of an animal, dissolved drug concentration may be determined by a conventional method known in the art. One method is a deconvolution method. In this method, the serum or plasma drug concentration is plotted along the ordinate (y-axis) against the blood sample time along the abscissa (x-axis). The data may then be analyzed to determine drug release rates in the GI tract using any conventional analysis, such as the Wagner-Nelson or Loo-Riegelman analysis. See also Welling, "Pharmacokinetics: Processes and Mathematics" (ACS Monograph 185, Amer. Chem. Soc., Washington, D.C., 1986). Treatment of the data in this manner yields an apparent *in vivo* drug release profile. Another method is to intubate the patient and periodically sample the GI tract directly.

The solid amorphous dispersions of CETP inhibitor and concentration-enhancing polymer used in the inventive dosage forms provide enhanced concentration of the dissolved CETP inhibitor in *in vitro* dissolution tests. It has been determined that enhanced drug concentration in *in vitro* dissolution tests in MFD solution or in PBS solution is a good indicator of *in vivo* performance and bioavailability. An appropriate PBS solution is an aqueous solution comprising 20 mM $Na_2HPO_4$, 47 mM $KH_2PO_4$, 87 mM NaCl, and 0.2 mM KCl, adjusted to pH 6.5 with NaOH. An appropriate MFD solution is the same PBS solution wherein there is also present 7.3 mM sodium taurocholic acid and 1.4 mM of 1-palmitoyl-2-oleyl-sn-glycero-3-phosphocholine. In particular, a composition formed by the inventive method can be dissolution-tested by adding it to MFD or PBS solution and agitating to promote dissolution.

An *in vitro* test to evaluate enhanced CETP inhibitor concentration in aqueous solution can be conducted by (1) adding with agitation a sufficient quantity of control composition, typically the undispersed CETP inhibitor alone, to the *in vitro* test medium, such as an MFD or a PBS solution, to achieve equilibrium concentration of the CETP inhibitor; (2) in a separate test, adding with agitation a sufficient quantity of test composition (e.g., the solid amorphous dispersion of CETP inhibitor and polymer or dosage form) in the same test medium, such that if all the CETP inhibitor dissolved, the theoretical concentration of CETP inhibitor would exceed the equilibrium concentration of the CETP inhibitor by a factor of at least 2, and preferably by a factor of at least 10; and (3) comparing the measured MDC and/or aqueous AUC of the test composition in the test medium with the equilibrium concentration, and/or with the aqueous AUC of the control composition. In conducting such a dissolution test, the amount of test composition or control composition used is an amount such that if all of the CETP inhibitor dissolved the CETP inhibitor concentration would be at least 2-fold, preferably at least 10-fold, and most preferably at least 100-fold that of the equilibrium concentration. Indeed, for some extremely insoluble CETP inhibitors, in order to identify the MDC achieved it may be necessary to use an amount of test composition such that if all of the CETP inhibitor dissolved, the CETP inhibitor concentration would be 1000-fold or even more, that of the equilibrium concentration of the CETP inhibitor.

The concentration of dissolved CETP inhibitor is typically measured as a function of time by sampling the test medium and plotting CETP inhibitor concentration in the test medium vs. time so that the MDC can be ascertained. The MDC is taken to be the maximum value of dissolved CETP inhibitor measured over the duration of the test. The aqueous AUC is calculated by integrating the concentration versus time curve over any 90-minute time period between the time of introduction of the composition into the aqueous use environment (when time equals zero) and 270 minutes following introduction to the use environment (when time equals 270 minutes). Typically, when the composition reaches its MDC rapidly, in say less than about 30 minutes, the time interval used to calculate AUC is from time equals zero to time equals 90 minutes. However, if the AUC of a composition over any 90-minute time period described above meets the criterion of this invention, then the composition formed is considered to be within the scope of this invention.

To avoid large drug particulates that would give an erroneous determination, the test solution is either filtered or centrifuged. "Dissolved drug" is typically taken as that material that either passes a 0.45 μm syringe filter or, alternatively, the material that remains in the supernatant following centrifugation. Filtration can be conducted using a 13 mm, 0.45 μm polyvinylidine difluoride syringe filter sold by Scientific Resources under the trademark TITAN®. Centrifugation is typically carried out in a polypropylene microcentrifuge tube by centrifuging at 13,000 G for 60 seconds. Other similar filtration or centrifugation methods can be employed and useful results obtained. For example, using other types of microfilters may yield values somewhat higher or lower (±10-40%) than that obtained with the filter specified above but

will still allow identification of preferred dispersions. It should be recognized that this definition of "dissolved drug" encompasses not only monomeric solvated drug molecules but also a wide range of species such as polymer/drug assemblies that have submicron dimensions such as drug aggregates, aggregates of mixtures of polymer and drug, micelles, polymeric micelles, colloidal particles or nanocrystals, polymer/drug complexes, and other such drug-containing species that are present in the filtrate or supernatant in the specified dissolution test.

In another separate aspect, the solid amorphous dispersions, when dosed orally to a human or other animal in a fasted state, provides improved concentration of dissolved CETP inhibitor in the blood relative to the control composition. The solid amorphous dispersion achieves a higher maximum drug concentration ($C_{max}$) of the CETP inhibitor in the blood (serum or plasma) relative to a control composition consisting of an equivalent amount of crystalline drug in its lowest energy form, or amorphous form if the crystalline form is unknown. It is to be understood that the control composition is free from solubilizers or other components that would materially affect the solubility of the CETP inhibitor. Preferably, the solid amorphous dispersion provides a $C_{max}$ of CETP inhibitor in the blood that is at least 1.25-fold that provided by the control composition, more preferably by at least 2-fold, and most preferably by at least 3-fold.

Alternatively, the solid amorphous dispersions, when dosed orally to a human or other animal, provide an AUC in CETP inhibitor concentration in the blood that is at least about 1.25-fold, preferably at least about 2-fold, preferably at least about 3-fold, preferably at least about 4-fold, preferably at least about 6-fold, preferably at least 10-fold, and even more preferably at least about 20-fold that observed when a control composition consisting of an equivalent quantity of undispersed CETP inhibitor is dosed. It is noted that such compositions can also be said to have a relative bioavailability of from about 1.25-fold to about 20-fold that of the control composition.

Relative bioavailability of CETP inhibitors in the solid amorphous dispersions or dosage forms can be tested *in vivo* in animals or humans using conventional methods for making such a determination. An *in vivo* test, such as a crossover study, may be used to determine whether a composition of CETP inhibitor and concentration-enhancing polymer provides an enhanced relative bioavailability compared with a control composition as described above. In an *in vivo* crossover study a test composition of a solid amorphous dispersion of a CETP inhibitor and polymer, or dosage form, is dosed to half a group of test subjects and, after an appropriate washout period (e.g., one week) the same subjects are dosed with a control composition. The other half of the group is dosed with the control composition first, followed by the test composition. The relative bioavailability is measured as the concentration in the blood (serum or plasma) versus time area under the curve (AUC) determined for the test group divided by the AUC in the blood provided by the control composition. Preferably, this test/control ratio is determined for each subject, and then the ratios are averaged over all subjects in the study. *In vivo* determinations of AUC can be made by plotting the serum or plasma concentration of drug along the ordinate (y-axis) against time along the abscissa (x-axis). To facilitate dosing, a dosing vehicle may be used to administer the dose. The dosing vehicle is preferably water, but may also contain materials for suspending the test or control composition, provided these materials do not dissolve the composition or change the drug solubility *in vivo.*

PREPARATION OF DISPERSIONS

The solid amorphous dispersions of CETP inhibitor and neutral or neutralized acidic polymer may be made according to any conventional process for forming solid amorphous dispersions that results in at least a major portion (at least 60%) of the CETP inhibitor being in the amorphous state. Such processes include mechanical, thermal and solvent processes. Exemplary mechanical processes include milling and extrusion; melt processes include high temperature fusion, solvent-modified fusion and melt-congeal processes; and solvent processes include non-solvent precipitation, spray-coating and spray-drying. See, for example, the following U.S. Patents, Nos. 5,456,923 and 5,939,099, which describe forming dispersions by extrusion processes; Nos. 5,340,591 and 4,673,564, which describe forming dispersions by milling processes; and Nos. 5,707,646 and 4,894,235, which describe forming dispersions by melt congeal processes.

When the CETP inhibitor has a relatively low melting point, typically less than about 200°C and preferably less than about 150°C, the use of a melt-congeal or melt-extrusion process is advantageous. In such processes, a molten mixture comprising the CETP inhibitor and concentration-enhancing polymer is rapidly cooled to solidify the molten mixture to form a solid amorphous dispersion. By "molten mixture" is meant that the mixture comprising the CETP inhibitor and concentration-enhancing polymer is heated sufficiently that it becomes sufficiently fluid that the CETP inhibitor substantially disperses in one or more of the concentration-enhancing polymers and other excipients. Generally, this requires that the mixture be heated to about 10°C or more above the melting point of the lowest melting excipient or CETP inhibitor in the composition. The CETP inhibitor may exist in the molten mixture as a pure phase, as a solution of CETP inhibitor homogeneously distributed throughout the molten mixture, or any combination of these states or those states that lie intermediate between them. The molten mixture is preferably substantially homogeneous so that the CETP inhibitor is dispersed as homogeneously as possible throughout the molten mixture. When the temperature of the molten mixture is below the melting point of both the CETP inhibitor and the concentration-enhancing polymer, the molten excipients, concentration-enhancing polymer, and CETP inhibitor are preferably sufficiently soluble in each other that a

substantial portion of the CETP inhibitor disperses in the concentration-enhancing polymer or excipients. It is often preferred that the mixture be heated above the lower of the melting points of the concentration-enhancing polymer and the CETP inhibitor. It should be noted that many concentration-enhancing polymers are amorphous. In such cases, melting point refers to the softening point of the polymer. Thus, although the term "melting point" generally refers specifically to the temperature at which a crystalline material transitions from its crystalline to its liquid state, as used herein, the term is used more broadly, referring to the heating of any material or mixture of materials sufficiently that it becomes fluid in a manner similar to a crystalline material in the fluid state.

Generally, the processing temperature may vary from 50°C up to about 200°C or higher, depending on the melting point of the CETP inhibitor and polymer, the latter being a function of the polymer grade selected. However, the processing temperature should not be so high that an unacceptable level of degradation of the CETP inhibitor or polymer occurs. In some cases, the molten mixture should be formed under an inert atmosphere to prevent degradation of the CETP inhibitor and/or polymer at the processing temperature. When relatively high temperatures are used, it is often preferable to minimize the time that the mixture is at the elevated temperature to minimize degradation.

The molten mixture may also include an excipient that will reduce the melting temperature of the molten mixture, thereby allowing processing at a lower temperature. When such excipients have low volatility and substantially remain in the mixture upon solidification, they generally can comprise up to 30 wt% of the molten mixture. For example, a plasticizer may be added to the mixture to reduce the melting temperature of the polymer. Examples of plasticizers include water, triethylcitrate, triacetin, and dibutyl sebacate. Volatile agents that dissolve or swell the polymer, such as acetone, water, methanol and ethyl acetate, may also be added to reduce the melting point of the molten mixture. When such volatile excipients are added, at least a portion, up to essentially all of such excipients may evaporate in the process of or following conversion of the molten mixture to a solid mixture. In such cases, the processing may be considered to be a combination of solvent processing and melt-congealing or melt-extrusion. Removal of such volatile excipients from the molten mixture can be accomplished by breaking up or atomizing the molten mixture into small droplets and contacting the droplets with a fluid so that the droplets both cool and lose all or part of the volatile excipient. Examples of other excipients that can be added to the mixture to reduce the processing temperature include low molecular weight polymers or oligomers, such as polyethylene glycol, polyvinylpyrrolidone, and poloxamers; fats and oils, including mono-, di-, and triglycerides; natural and synthetic waxes, such as Carnauba wax, beeswax, microcrystalline wax, castor wax, and paraffin wax; long chain alcohols, such as cetyl alcohol and stearyl alcohol; and long chain fatty acids, such as stearic acid. As mentioned above, when the excipient added is volatile, it may be removed from the mixture while still molten or following solidification to form the solid amorphous dispersion.

Virtually any process may be used to form the molten mixture. One method involves melting the concentration-enhancing polymer in a vessel and then adding the CETP inhibitor to the molten polymer. Another method involves melting the CETP inhibitor in a vessel and then adding the concentration-enhancing polymer. In yet another method, a solid blend of the CETP inhibitor and concentration-enhancing polymer may be added to a vessel and the blend heated to form the molten mixture.

Once the molten mixture is formed, it may be mixed to ensure the CETP inhibitor is homogeneously distributed throughout the molten mixture. Such mixing may be done using mechanical means, such as overhead mixers, magnetically driven mixers and stir bars, planetary mixers, and homogenizers. Optionally, when the molten mixture is formed in a vessel, the contents of the vessel can be pumped out of the vessel and through an in-line or static mixer and then returned to the vessel. The amount of shear used to mix the molten mixture should be sufficiently high to ensure uniform distribution of the CETP inhibitor in the molten mixture. The molten mixture can be mixed from a few minutes to several hours, the mixing time depending on the viscosity of the mixture and the solubility of the CETP inhibitor and the presence of optional excipients in the concentration-enhancing polymer.

Yet another method of preparing the molten mixture is to use two vessels, melting the CETP inhibitor in the first vessel and the concentration-enhancing polymer in a second vessel. The two melts are then pumped through an in-line static mixer or extruder to produce the molten mixture that is then rapidly solidified.

Still another method of preparing the molten mixture is by the use of an extruder, such as a single-screw or twin-screw extruder, both well known in the art. In such devices, a solid feed of the composition is fed to the extruder, whereby the combination of heat and shear forces produce a uniformly mixed molten mixture, which can then be rapidly solidified to form the solid amorphous dispersion. The solid feed can be prepared using methods well known in the art for obtaining solid mixtures with high content uniformity. Alternatively, the extruder may be equipped with two feeders, allowing the CETP inhibitor to be fed to the extruder through one feeder and the polymer through the other. Other excipients to reduce the processing temperature as described above may be included in the solid feed, or in the case of liquid excipients, such as water, may be injected into the extruder using methods well known in the art.

The extruder should be designed so that it produces a molten mixture with the CETP inhibitor uniformly distributed throughout the composition. Various zones in the extruder should be heated to appropriate temperatures to obtain the desired extrudate temperature as well as the desired degree of mixing or shear, using procedures well known in the art.

When the CETP inhibitor has a high solubility in the concentration-enhancing polymer, a lower amount of mechanical energy will be required to form the solid amorphous dispersion. In the case where the melting point of the undispersed CETP inhibitor is greater than the melting point of the undispersed concentration-enhancing polymer, the processing temperature may be below the melting temperature of the undispersed CETP inhibitor but greater than the melting point of the polymer, since the CETP inhibitor will dissolve into the molten polymer. When the melting point of the undispersed CETP inhibitor is less than the melting point of the undispersed concentration-enhancing polymer, the processing temperature may be above the melting point of the undispersed CETP inhibitor but below the melting point of the undispersed concentration-enhancing polymer since the molten CETP inhibitor will dissolve in or be absorbed into the polymer.

When the CETP inhibitor has a low solubility in the polymer, a higher amount of mechanical energy may be required to form the solid amorphous dispersion. Here, the processing temperature may need to be above the melting point of the CETP inhibitor and the polymer. As mentioned above, alternatively, a liquid or low-melting point excipient may be added that promotes melting or the mutual solubility of the concentration-enhancing polymer and a CETP inhibitor. A high amount of mechanical energy may also be needed to mix the CETP inhibitor and the polymer to form a dispersion. Typically, the lowest processing temperature and an extruder design that imparts the lowest amount of mechanical energy, *i.e.*, shear, that produces a satisfactory dispersion (substantially amorphous and substantially homogeneous) is chosen in order to minimize the exposure of the CETP inhibitor to harsh conditions.

Once the molten mixture of CETP inhibitor and concentration-enhancing polymer is formed, the mixture should be rapidly solidified to form the solid amorphous dispersion. By "rapidly solidified" is meant that the molten mixture is solidified sufficiently fast that substantial phase separation of the CETP inhibitor and polymer does not occur. Typically, this means that the mixture should be solidified in less than about 10 minutes, preferably less than about 5 minutes and more preferably less than about 1 minute. If the mixture is not rapidly solidified, phase separation can occur, resulting in the formation of CETP inhibitor-rich and polymer-rich phases.

Solidification often takes place primarily by cooling the molten mixture to at least about 10°C and preferably at least about 30°C below it's melting point. As mentioned above, solidification can be additionally promoted by evaporation of all or part of one or more volatile excipients or solvents. To promote rapid cooling and evaporation of volatile excipients, the molten mixture is often formed into a high surface area shape such as a rod or fiber or droplets. For example, the molten mixture can be forced through one or more small holes to form long thin fibers or rods or may be fed to a device, such as an atomizer such as a rotating disk, that breaks the molten mixture up into droplets from 1 μm to 1 cm in diameter. The droplets are then contacted with a relatively cool fluid such as air or nitrogen to promote cooling and evaporation.

A useful tool for evaluating and selecting conditions for forming substantially homogeneous, substantially amorphous dispersions via a melt-congeal or melt-extrusion process is the differential scanning calorimeter (DSC). While the rate at which samples can be heated and cooled in a DSC is limited, it does allow for precise control of the thermal history of a sample. For example, the CETP inhibitor and concentration-enhancing polymer may be dry-blended and then placed into the DSC sample pan. The DSC can then be programmed to heat the sample at the desired rate, hold the sample at the desired temperature for a desired time, and then rapidly cool the sample to ambient or lower temperature. The sample can then be re-analyzed on the DSC to verify that it was transformed into a substantially homogeneous, substantially amorphous dispersion (*i.e.*, the sample has a single Tg). Using this procedure, the temperature and time required to achieve a substantially homogeneous, substantially amorphous dispersion for a given CETP inhibitor and concentration-enhancing polymer can be determined.

Another method for forming solid amorphous dispersions is by "solvent processing," which consists of dissolution of the CETP inhibitor and one or more polymers in a common solvent. "Common" here means that the solvent, which can be a mixture of compounds, will dissolve both the CETP inhibitor and the polymer(s). After both the CETP inhibitor and the polymer have been dissolved, the solvent is rapidly removed by evaporation or by mixing with a non-solvent. Exemplary processes are spray-drying, spray-coating (pan-coating, fluidized bed coating, etc.), and precipitation by rapid mixing of the polymer and CETP inhibitor solution with $CO_2$, water, or some other non-solvent. Preferably, removal of the solvent results in the formation of a substantially homogeneous, solid amorphous dispersion. In such dispersions, the CETP inhibitor is dispersed as homogeneously as possible throughout the polymer and can be thought of as a solid solution of CETP inhibitor dispersed in the polymer(s), wherein the solid amorphous dispersion is thermodynamically stable, meaning that the concentration of CETP inhibitor in the polymer is at or below its equilibrium value, or it may be considered to be a supersaturated solid solution where the CETP inhibitor concentration in the concentration-enhancing polymer(s) is above its equilibrium value.

The solvent may be removed by spray-drying. The term "spray-drying" is used conventionally and broadly refers to processes involving breaking up liquid mixtures into small droplets (atomization) and rapidly removing solvent from the mixture in a spray-drying apparatus where there is a strong driving force for evaporation of solvent from the droplets. Spray-drying processes and spray-drying equipment are described generally in Perry's Chemical Engineers' Handbook, pages 20-54 to 20-57 (Sixth Edition 1984). More details on spray-drying processes and equipment are reviewed by Marshall, "Atomization and Spray-Drying," 50 Chem. Eng. Prog. Monogr. Series 2 (1954), and Masters,

Spray Drying Handbook (Fourth Edition 1985). The strong driving force for solvent evaporation is generally provided by maintaining the partial pressure of solvent in the spray-drying apparatus well below the vapor pressure of the solvent at the temperature of the drying droplets. This is accomplished by (1) maintaining the pressure in the spray-drying apparatus at a partial vacuum (*e.g.*, 0.01 to 0.50 atm); or (2) mixing the liquid droplets with a warm drying gas; or (3) both (1) and (2). In addition, at least a portion of the heat required for evaporation of solvent may be provided by heating the spray solution.

Solvents suitable for spray-drying can be any organic compound in which the CETP inhibitor and polymer are mutually soluble. Preferably, the solvent is also volatile with a boiling point of 150°C or less. In addition, the solvent should have relatively low toxicity and be removed from the solid amorphous dispersion to a level that is acceptable according to The International Committee on Harmonization (ICH) guidelines. Removal of solvent to this level may require a subsequent processing step such as tray-drying. Preferred solvents include alcohols such as methanol, ethanol, n-propanol, iso-propanol, and butanol; ketones such as acetone, methyl ethyl ketone and methyl iso-butyl ketone; esters such as ethyl acetate and propylacetate; and various other solvents such as acetonitrile, methylene chloride, toluene, and 1,1,1-trichloroethane. Lower volatility solvents such as dimethyl acetamide or dimethylsulfoxide can also be used. Mixtures of solvents, such as 50% methanol and 50% acetone, can also be used, as can mixtures with water, so long as the polymer and CETP inhibitor are sufficiently soluble to make the spray-drying process practicable. Generally, due to the hydrophobic nature of low-solubility CETP inhibitors, non-aqueous solvents are preferred, meaning that the solvent comprises less than about 10 wt% water.

The solvent-bearing feed, comprising the CETP inhibitor and the concentration-enhancing polymer, can be spray-dried under a wide variety of conditions and yet still yield dispersions with acceptable properties. For example, various types of nozzles can be used to atomize the spray solution, thereby introducing the spray solution into the spray-dry chamber as a collection of small droplets. Essentially any type of nozzle may be used to spray the solution as long as the droplets that are formed are sufficiently small that they dry sufficiently (due to evaporation of solvent) that they do not stick to or coat the spray-drying chamber wall.

Although the maximum droplet size varies widely as a function of the size, shape and flow pattern within the spray-dryer, generally droplets should be less than about 500 μm in diameter when they exit the nozzle. Examples of types of nozzles that may be used to form the solid amorphous dispersions include the two-fluid nozzle, the fountain-type nozzle, the flat fan-type nozzle, the pressure nozzle and the rotary atomizer. In a preferred embodiment, a pressure nozzle is used, as disclosed in detail in commonly assigned copending U.S. Provisional Application No. 60/353,986.

The spray solution can be delivered to the spray nozzle or nozzles at a wide range of temperatures and flow rates. Generally, the spray solution temperature can range anywhere from just above the solvent's freezing point to about 20°C above its ambient pressure boiling point (by pressurizing the solution) and in some cases even higher. Spray solution flow rates to the spray nozzle can vary over a wide range depending on the type of nozzle, spray-dryer size and spray-dry conditions such as the inlet temperature and flow rate of the drying gas. Generally, the energy for evaporation of solvent from the spray solution in a spray-drying process comes primarily from the drying gas.

The drying gas can, in principle, be essentially any gas, but for safety reasons and to minimize undesirable oxidation of the CETP inhibitor or other materials in the solid amorphous dispersion, an inert gas such as nitrogen, nitrogen-enriched air or argon is utilized. The drying gas is typically introduced into the drying chamber at a temperature between about 60° and about 300°C and preferably between about 80° and about 240°C.

The large surface-to-volume ratio of the droplets and the large driving force for evaporation of solvent leads to rapid solidification times for the droplets. Solidification times should be less than about 20 seconds, preferably less than about 10 seconds, and more preferably less than 1 second. This rapid solidification is often critical to the particles maintaining a uniform, homogeneous dispersion instead of separating into CETP inhibitor-rich and polymer-rich phases. In a preferred embodiment, the height and volume of the spray-dryer are adjusted to provide sufficient time for the droplets to dry prior to impinging on an internal surface of the spray-dryer, as described in detail in commonly assigned, copending U.S. Provisional Application No. 60/354,080. As noted above, to get large enhancements in concentration and bioavailability it is often necessary to obtain as homogeneous a dispersion as possible.

Following solidification, the solid powder typically stays in the spray-drying chamber for about 5 to 60 seconds, further evaporating solvent from the solid powder. The final solvent content of the solid dispersion as it exits the dryer should be low, since this reduces the mobility of the CETP inhibitor molecules in the solid amorphous dispersion, thereby improving its stability. Generally, the solvent content of the solid amorphous dispersion as it leaves the spray-drying chamber should be less than 10 wt% and preferably less than 2 wt%. Following formation, the solid amorphous dispersion can be dried to remove residual solvent using suitable drying processes, such as tray drying, fluid bed drying, microwave drying, belt drying, rotary drying, and other drying processes known in the art.

The solid amorphous dispersion is usually in the form of small particles. The mean size of the particles may be less than 500 μm in diameter, or less than 100 μm in diameter, less than 50 μm in diameter or less than 25 μm in diameter. When the solid amorphous dispersion is formed by spray-drying, the resulting dispersion is in the form of such small particles. When the solid amorphous dispersion is formed by other methods such as by melt-congeal or extrusion

processes, the resulting solid amorphous dispersion may be sieved, ground, or otherwise processed to yield a plurality of small particles.

Once the solid amorphous dispersion comprising the CETP inhibitor and concentration-enhancing polymer has been formed, several processing operations can be used to facilitate incorporation of the solid amorphous dispersion into a dosage form. These processing operations include drying, granulation, and milling.

The solid amorphous dispersion may be granulated to increase particle size and improve handling of the solid amorphous dispersion while forming a suitable dosage form. Preferably, the average size of the granules will range from 50 to 1000 μm. Such granulation processes may be performed before or after the composition is dried, as described above. Dry or wet granulation processes can be used for this purpose. An example of a dry granulation process is roller compaction. Wet granulation processes can include so-called low shear and high shear granulation, as well as fluid bed granulation. In these processes, a granulation fluid is mixed with the composition after the dry components have been blended to aid in the formation of the granulated composition. Examples of granulation fluids include water, ethanol, isopropyl alcohol, n-propanol, the various isomers of butanol, and mixtures thereof.

If a wet granulation process is used, the granulated composition is often dried prior to further processing. Examples of suitable drying processes to be used in connection with wet granulation are the same as those described above. Where the solid amorphous dispersion is made by a solvent process, the composition can be granulated prior to removal of residual solvent. During the drying process, residual solvent and granulation fluid are concurrently removed from the composition.

Once the composition has been granulated, it may then be milled to achieve the desired particle size. Examples of suitable processes for milling the composition include hammer milling, ball milling, fluid-energy milling, roller milling, cutting milling, and other milling processes known in the art.

Processes for forming solid amorphous dispersions of CETP inhibitors and concentration-enhancing polymers are described in detail in commonly assigned, copending U.S. Patent Application Nos. 09/918,127 and 10/066,091.

## HMG-CoA REDUCTASE INHIBITORS

The HMG-CoA reductase inhibitor may be any HMG-CoA reductase inhibitor capable of lowering plasma concentrations of low-density lipoprotein, total cholesterol, or both. The HMG-CoA reductase inhibitor is acid-sensitive, meaning that the drug either chemically reacts with or otherwise degrades in the presence of acidic species. Examples of chemical reactions include hydrolysis, lactonization, or transesterification in the presence of acidic species.

In one aspect, the HMG-CoA reductase inhibitor is from a class of therapeutics commonly called statins. Examples of HMG-CoA reductase inhibitors that may be used include but are not limited to lovastatin (MEVACOR®; see U.S. Pat. Nos. 4,231,938; 4,294,926; 4,319,039), simvastatin (ZOCOR®; see U.S. Pat. Nos. 4,444,784; 4,450,171, 4,820,850; 4,916,239), pravastatin (PRAVACHOL®; see U.S. Pat. Nos. 4,346,227; 4,537,859; 4,410,629; 5,030,447 and 5,180,589), lactones of pravastatin (see U.S. Pat. No. 4,448,979), fluvastatin (LESCOL®; see U.S. Pat. Nos. 5,354,772; 4,911,165; 4,739,073; 4,929,437; 5,189,164; 5,118,853; 5,290,946; 5,356,896), lactones of fluvastatin, atorvastatin (LIPITOR®; see U.S. Pat: Nos. 5,273,995; 4,681,893; 5,489,691; 5,342,952), lactones of atorvastatin, cerivastatin (also known as rivastatin and BAYCHOL®; see U.S. Pat. No. 5,177,080 and European Application No. EP-491226A), lactones of cerivastatin, rosuvastatin (Crestor®; see U.S. Pat. Nos. 5,260,440 and RE37314, and European Patent No. EP521471), lactones of rosuvastatin, itavastatin, nisvastatin, visastatin, atavastatin, bervastatin, compactin, dihydrocompactin, dalvastatin, fluindostatin, pitivastatin, mevastatin (see U.S. Pat. No. 3,983,140), and velostatin (also referred to as synvinolin). Other examples of HMG-CoA reductase inhibitors are described in U.S. Pat. Nos. 5,217,992; 5,196,440; 5,189,180; 5,166,364; 5,157,134; 5,110,940; 5,106,992; 5,099,035; 5,081,136; 5,049,696; 5,049,577; 5,025,017; 5,011,947; 5,010,105; 4,970,221; 4,940,800; 4,866,058; 4,686,237; 4,647,576; European Application Nos. 0142146A2 and 0221025A1; and PCT Application Nos. WO 86/03488, WO 86/07054, and WO 97/06802. Also included are pharmaceutically acceptable forms of the above. Preferably the HMG-CoA reductase inhibitor is selected from the group consisting of fluvastatin, lovastatin, pravastatin, atorvastatin, simvastatin, cerivastatin, rivastatin, mevastatin, velostatin, compactin, dalvastatin, fluindostatin, rosuvastatin, pitivastatin, dihydrocompactin, and pharmaceutically acceptable forms thereof. By "pharmaceutically acceptable forms" is meant any pharmaceutically acceptable derivative or variation, including stereoisomers, stereoisomer mixtures, enantiomers, solvates, hydrates, isomorphs, polymorphs, salt forms and prodrugs.

A test to determine whether an HMG-CoA reductase inhibitor is acid sensitive is to administer the drug to an acidic aqueous solution and plot drug concentration versus time. The acidic solution should have a pH of from 1-4. HMG-CoA reductase inhibitors that are acid sensitive are those for which the drug concentration decreases by at least 1% within 24 hours of administration of the drug to the acidic solution. If the drug concentration changes by 1 % in the 6-24 hour time period, then the drug is "slightly acid-sensitive." If the drug concentration changes by 1% in the 1-6 hour time period, then the drug is "moderately acid-sensitive." If the drug concentration changes by 1 % in less than 1 hour, then the drug is "highly acid-sensitive." The present invention finds increasing utility for HMG-CoA reductase inhibitors that

are slightly acid-sensitive, moderately acid-sensitive and highly acid-sensitive.

In one embodiment, the HMG-CoA reductase inhibitor is selected from the group consisting of trans-6-[2-(3 or 4-carboxamido-substituted pyrrol-1-yl)alkyl]-4-hydroxypyran-2-ones and corresponding pyran ring-opened hydroxy acids derived therefrom. These compounds have been described in U.S. Pat. No. 4,681,893. The pyran ring-opened hydroxy acids which are intermediates in the synthesis of the lactone compounds can be used as free acids or as pharmaceutically acceptable metal or amine salts. In particular, these compounds can be represented by the following structure:

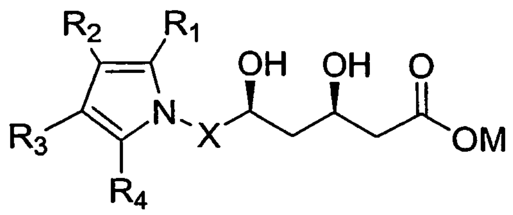

wherein X is $--CH_2--$, $--CH_2CH_2--$, $--CH_2CH_2CH_2--$ or $--CH_2CH(CH_3)--$;

$R_1$ is 1-naphthyl; 2-naphthyl; cyclohexyl, norbornenyl; 2-,3-, or 4-pyridinyl; phenyl; phenyl substituted with fluorine, chlorine, bromine, hydroxyl, trifluoromethyl, alkyl of from one to four carbon atoms, alkoxy of from one to four carbon atoms, or alkanoylalkoxy of from two to eight carbon atoms; either $R_2$ or $R_3$ is $--CONR_5R_6$ where $R_5$ and $R_6$ are independently hydrogen; alkyl of from one to six carbon atoms; 2-,3-, or 4-pyridinyl; phenyl; phenyl substituted with fluorine, chlorine, bromine, cyano, trifluoromethyl, or carboalkoxy of from three to eight carbon atoms; and the other of $R_2$ or $R_3$ is hydrogen; alkyl of from one to six carbon atoms; cyclopropyl; cyclobutyl; cyclopentyl; cyclohexyl; phenyl; or phenyl substituted with fluorine, chlorine, bromine, hydroxyl, trifluoromethyl, alkyl of from one to four carbon atoms, alkoxy of from one to four carbon atoms, or alkanoyloxy of from two to eight carbon atoms; $R_4$ is alkyl of from one to six carbon atoms; cyclopropyl; cyclobutyl; cyclopentyl; cyclohexyl; or trifluoromethyl; and M is a pharmaceutically acceptable salt (e.g., counter ion), which includes a pharmaceutically acceptable metal salt or a pharmaceutically acceptable amine salt.

Among the stereo-specific isomers, one preferred HMG-CoA reductase inhibitor is atorvastatin trihydrate hemicalcium salt. This preferred compound is the ring-opened form of (2R-trans)-5-(4-fluorophenyl)-2-(1 methylethyl)-N,4-diphenyl-1-[2-(tetrahydro-4-hydroxy-6-oxo-2H-pyran-2-yl)ethyl]-1H-pyrrole-3-carboxamide, namely, the enantiomer [R-(R*,R*)]-2-(4-fluorophenyl-β, δ-dihydroxy-5-(1-methylethyl)-3 - phenyl-4-[(phenylamino)carbonyl)]-1H-pyrrole-1-heptanoic acid hemicalcium salt. Its chemical structure may be represented by the following structure:

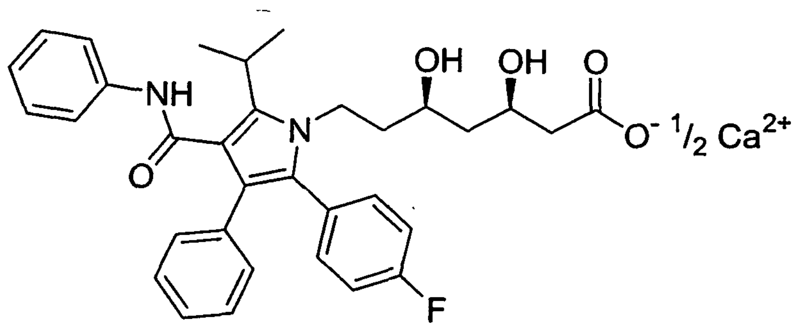

Formula A

The specific isomer has been described in U.S. Pat. No. 5,273,995. In a preferred embodiment, the HMG-CoA reductase inhibitor is selected from the group consisting of atorvastatin, the cyclized lactone form of atorvastatin, a 2-hydroxy, 3-hydroxy or 4-hydroxy derivative of such compounds, and pharmaceutically acceptable forms thereof.

In practice, use of the salt form amounts to use of the acid or lactone form. Appropriate pharmaceutically acceptable salts within the scope of the invention are those derived from bases such as sodium hydroxide, potassium hydroxide, lithium hydroxide, calcium hydroxide, 1-deoxy-2-(methylamino)-D-glucitol, magnesium hydroxide, zinc hydroxide, aluminum hydroxide, ferrous or ferric hydroxide, ammonium hydroxide or organic amines such as N-methylglucamine, choline, arginine and the like. Preferably, the lithium, calcium, magnesium, aluminum and ferrous or ferric salts are prepared from the sodium or potassium salt by adding the appropriate reagent to a solution of the sodium or potassium salt, i.e., addition of calcium chloride to a solution of the sodium or potassium salt of the compound of the formula A will give the calcium salt thereof.

PREPARATION OF UNITARY DOSAGE FORMS

The unitary dosage form may be in the form of a tablet, caplet, pill, capsule, powder or other dosage form known in the art. The amount of CETP inhibitor and HMG-CoA reductase inhibitor present in the dosage form will vary depending on the desired dose for each compound, which in turn, depends on the potency of the compound and the condition being treated. For example, the desired dose for the CETP inhibitor torcetrapib, also known as [2R,4S]-4-[(3,5-bis-trifluoromethyl-benzyl)-methoxycarbonyl-amino]-2-ethyl-6-trifluoromethyl-3,4-dihydro-2H-quinoline-1-carboxylic acid ethyl ester, ranges from 1 mg/day to 1000 mg/day, preferably 10 to 250 mg/day, more preferably 30 to 90 mg/day. For the HMG-CoA reductase inhibitor atorvastatin calcium, the dose ranges from 1 to 160 mg/day, preferably 2 to 80 mg/day. For the HMG-CoA reductase inhibitors lovastatin, pravastatin sodium, simvastatin, rosuvastatin calcium, and fluvastatin sodium, the dose ranges from 2 to 160 mg/day, preferably 10 to 80 mg/day. For the HMG-CoA reductase inhibitor cerivastatin sodium, the dose ranges from 0.05 to 1.2 mg/day, preferably 0.1 to 1.0 mg/day.
One method for forming the unitary dosage form is to first blend the solid amorphous dispersion of the CETP inhibitor and neutral or neutralized concentration-enhancing polymer, the HMG-CoA reductase inhibitor, and optional excipients using procedures well-known in the art. See for example, Remington: The Science and Practice of Pharmacy, 20th Edition (2000). Examples of blending equipment include twin-shell blenders, fluidized beds, and V blenders.
Alternatively, the CETP inhibitor/polymer solid amorphous dispersion, HMG-CoA reductase inhibitor, and optional excipients, may be granulated. Exemplary methods for granulating the materials are wet granulation and dry granulation, both well known in the art. For example, the solid amorphous dispersion, HMG-CoA reductase inhibitor, and optional excipients may be granulated by mechanical means by, for example, roller compaction or "slugging," followed by milling to form granules. The granules typically have improved flow, handling, blending, and compression properties relative to the ungranulated materials. Wet granulation techniques may also be employed, provided the solvents and process selected do not alter the properties of the solid amorphous dispersion. When wet granulation is used, the granulation liquid is typically removed from the granules during or after the granulation process. The so-formed granules typically have an average diameter ranging from 50 μm to 1000 μm, preferably 50 μm to about 800 μm, although granules outside this range can be used. Improved wetting, disintegrating, dispersing and dissolution properties may be obtained by the inclusion of other excipients described below.
Other conventional formulation excipients may be employed in the dosage form, including those excipients well known in the art, e.g., as described in Remington: The Science and Practice of Pharmacy, 20th Edition (2000). Generally, excipients such as surfactants, pH modifiers, disintegrants, porosigens, fillers, matrix materials, complexing agents, solubilizers, pigments, lubricants, glidants, flavorants, antioxidants, and so forth may be used for customary purposes and in typical amounts without adversely affecting the properties of the compositions.
One very useful class of excipients is surfactants, preferably present from 0 to 10 wt%. Suitable surfactants include fatty acid and alkyl sulfonates; commercial surfactants such as benzalkonium chloride (HYAMINE® 1622 from Lonza, Inc. of Fairlawn, New Jersey); dioctyl sodium sulfosuccinate (DOCUSATE SODIUM from Mallinckrodt Specialty Chemicals of St. Louis, Missouri); polyoxyethylene sorbitan fatty acid esters (TWEEN® from ICI Americas Inc. of Wilmington, Delaware; LIPOSORB® O-20 from Lipochem Inc. of Patterson New Jersey; CAPMUL® POE-0 from Abitec Corp. of Janesville, Wisconsin); natural surfactants such as sodium taurocholic acid, 1-palmitoyl-2-oleoyl-sn-glycero-3-phosphocholine, lecithin, and other phospholipids and mono- and diglycerides; and polyoxyethylene-polyoxypropylene . Such materials can advantageously be employed to increase the rate of dissolution by, for example, facilitating wetting, or otherwise increase the rate of release of the CETP inhibitor and/or the HMG-CoA reductase inhibitor from the dosage form.
Inclusion of pH modifiers such as acids, bases, or buffers may also be beneficial in an amount of from 0 to 10 wt%. In a preferred embodiment, the unitary dosage form also includes a base. The inclusion of a base can locally raise the pH in the dosage form, leading to an improvement in chemical stability of the HMG-CoA reductase inhibitor. The term "base" is used broadly to include not only strong bases such as sodium hydroxide, but also weak bases and buffers that are capable of achieving the desired increase chemical stability. Examples of bases include hydroxides, such as sodium hydroxide, calcium hydroxide, ammonium hydroxide, and choline hydroxide; bicarbonates, such as sodium bicarbonate, potassium bicarbonate, and ammonium bicarbonate; carbonates, such as ammonium carbonate, calcium carbonate, and sodium carbonate; amines, such as tris(hydroxymethyl)amino methane, ethanolamine, diethanolamine, N-methyl glucamine, glucosamine, ethylenediamine, N,N'-dibenzylethylenediamine, N-benzyl-2-phenethylamine, cyclohexylamine, cyclopentylamine, diethylamine, isopropylamine, diisopropylamine, dodecylamine, and triethylamine; proteins, such as gelatin; amino acids such as lysine, arginine, guanine, glycine, and adenine; polymeric amines, such as polyamino methacrylates, such as Eudragit E; conjugate bases of various acids, such as sodium acetate, sodium

benzoate, ammonium acetate, disodium phosphate, trisodium phosphate, calcium hydrogen phosphate, sodium phenolate, sodium sulfate, ammonium chloride, and ammonium sulfate; salts of EDTA, such as tetra sodium EDTA; and salts of various acidic polymers such as sodium starch glycolate, sodium carboxymethyl cellulose and sodium polyacrylic acid. In one embodiment, the base partially neutralizes the acidic concentration-enhancing polymer, as previously discussed; however, a base may also be included when the solid amorphous dispersion comprises a CETP inhibitor and a neutral concentration-enhancing polymer.

Examples of disintegrants include sodium starch glycolate, sodium carboxymethyl cellulose, calcium carboxymethyl cellulose, croscarmellose sodium, crospovidone, polyvinylpolypyrrolidone, methyl cellulose, microcrystalline cellulose, powdered cellulose, lower alkyl-substituted hydroxypropyl cellulose, polacrilin potassium, starch, pregelatinized starch, sodium alginate, and mixtures thereof. Generally, the disintegrant will comprise from 1 wt% to 25 wt%, preferably from 5 wt% to 20 wt% of the dosage form.

The unitary dosage form may also include a porosigen. A "porosigen" is a material that, when present in the formulation containing the solid amorphous dispersion, leads to a high porosity and high strength following compression of the blend into a tablet. In addition, preferred porosigens are soluble in an acidic environment with aqueous solubilities typically greater than 1 mg/mL at a pH less than about 4. Generally, the predominant deformation mechanism for porosigens under compression is brittle fracture rather than plastic flow. Examples of porosigens include acacia, calcium carbonate, calcium sulfate, calcium sulfate dihydrate, compressible sugar, dibasic calcium phosphate (anhydrous and dihydrate), tribasic calcium phosphate, monobasic sodium phosphate, dibasic sodium phosphate, lactose, magnesium oxide, magnesium carbonate, silicon dioxide, magnesium aluminum silicate, maltodextrin, mannitol, methyl cellulose, microcrystalline cellulose, sorbitol, sucrose and xylitol. Of these, microcrystalline cellulose and both forms of dibasic calcium phosphate (anhydrous and dihydrate) are preferred. Generally, the porosigen will comprise from 5 to 70 wt%, and preferably from 10 to 50 wt% of the dosage form.

Examples of other matrix materials, fillers, or diluents include dextrose, compressible sugar, hydrous lactose, corn starch, silicic anhydride, polysaccharides, dextrates, dextran, dextrin, dextrose, calcium carbonate, calcium sulfate, poloxamers, and polyethylene oxide.

Another optional excipient is a binder such as methyl cellulose, carboxymethylcellulose, hydroxypropylcellulose, hydroxymethylpropylcellulose, polyvinylpyrrolidone, polyvinylalcohol or starch.

Examples of drug-complexing agents or solubilizers include polyethylene glycols, caffeine, xanthene, gentisic acid and cylodextrins.

Examples of lubricants include calcium stearate, glyceryl monostearate, glyceryl palmitostearate, hydrogenated vegetable oil, light mineral oil, magnesium stearate, mineral oil, polyethylene glycol, sodium benzoate, sodium lauryl sulfate, sodium stearyl fumarate, stearic acid, talc and zinc stearate.

Examples of glidants include silicon dioxide, talc and cornstarch.

Examples of antioxidants include butylated hydroxyanisole, sodium ascorbate, butylated hydroxytoluene, sodium metabisulfate, malic acid, citric acid and ascorbic acid.

In a preferred embodiment, the unitary dosage form comprises a stabilizing agent for the HMG-CoA reductase inhibitor. The stabilizing agent stabilizes the HMG-CoA reductase inhibitor by reducing acid catalyzed degradation. The stabilizing agent may be a basic inorganic pharmaceutically acceptable salt. Exemplary salts include: salts of calcium, such as calcium carbonate and calcium hydroxide; salts of magnesium, such as magnesium carbonate, magnesium hydroxide, magnesium oxide, magnesium silicate, magnesium aluminate, and aluminum magnesium hydroxide; salts of lithium, such as lithium hydroxide and similar lithium compounds; or, other similarly suitable salts of alkaline earth metals. The basic inorganic salts of calcium, lithium or magnesium can be utilized in a weight ratio ranging between about 0.1 to 1 and about 50 to 1 of salt compound to HMG-CoA reductase inhibitor.

A preferred stabilizing agent is calcium carbonate. The inventors have observed that the size of the calcium carbonate particles is related to the effectiveness of the calcium carbonate as a stabilizing agent, with smaller particle size resulting in better performance as a stabilizing agent. Preferred grades of calcium carbonate are precipitated grades of calcium carbonate having a particle size of less than about ten microns ($\mu$m). Exemplary grades of precipitated calcium carbonate include Vicality Medium PCC and Vicality Heavy PCC available from Specialty Minerals, Pre-carb 150 available from Mutchler, and PCC-250 available from Particle Dynamics.

The CETP inhibitor/polymer solid amorphous dispersion, HMG-CoA reductase inhibitor, and optional excipients may be blended or granulated and then compressed to form the dosage form, such as tablets, caplets, or pills. The compressed dosage forms may be formed using any of a wide variety of presses used in the fabrication of pharmaceutical dosage forms. Examples include single-punch presses, rotary tablet presses, and multilayer rotary tablet presses, all well-known in the art. See Remington: The Science and Practice of Pharmacy (20th Edition, 2000). The compressed dosage form may be of any shape, including round, oval, oblong, cylindrical, or triangular. The upper and lower surfaces of the compressed dosage form may be flat, round, concave, or convex.

When formed by compression, the dosage form preferably has a "strength" of at least 5 Kiloponds (Kp)/$cm^2$, and more preferably at least 7 Kp/$cm^2$. Here, "strength" is the fracture force, also known as the tablet "hardness," required

to fracture a tablet formed from the materials, divided by the maximum cross-sectional area of the tablet normal to that force. The fracture force may be measured using a Schleuniger Tablet Hardness Tester, model 6D. To achieve the desired strength, the blend of the CETP inhibitor/polymer solid amorphous dispersion, HMG-CoA reductase inhibitor, and optional excipients should be compressed with sufficient force while forming the dosage form. The compression force required to achieve this strength will depend on the size of the tablet, but generally will be greater than about 5 $kP/cm^2$. Friability is a well-known measure of a dosage form's resistance to surface abrasion that measures weight loss in percentage after subjecting the dosage form to a standardized agitation procedure. Friability values of from 0.8 to 1.0% are regarded as constituting the upper limit of acceptability. Dosage forms having a strength of greater than 5 $kP/cm^2$ generally are very robust, having a friability of less than 0.5%, preferably less than 0.1%.

Alternatively, the CETP inhibitor/polymer solid amorphous dispersion, HMG-CoA reductase inhibitor, and optional excipients described above may be filled into a capsule, such as a hard- or soft-gelatin capsule or a capsule made from some other material, e.g., starch, to form the unitary dosage form. Such capsules are well known in the art (see, for example, Remington: The Science and Practice of Pharmacy (20th Edition, 2000)).

Yet another embodiment of the unitary dosage form is a powder form. The powder dosage form can then be taken dry or mixed with a liquid to form a paste, suspension or slurry prior to dosing. An example of this type of dosage form is a sachet, sometimes known in the art as an oral powder for constitution (OPC). The CETP inhibitor/polymer solid amorphous dispersion, the HMG-CoA reductase inhibitor, and optional excipients may be mixed and placed into a suitable container, such as a pouch, bottle, box, bag, or other container known in the art.

In another embodiment, the unitary dosage form comprises (a) a CETP inhibitor composition, the CETP inhibitor composition comprising a solid amorphous dispersion of a CETP inhibitor and a neutral or neutralized concentration-enhancing polymer; and (b) an HMG-CoA reductase inhibitor composition comprising an HMG-CoA reductase inhibitor. The CETP inhibitor composition and the HMG-CoA reductase inhibitor composition are combined such that the solid amorphous dispersion and the HMG-CoA reductase inhibitor are substantially separate from one another in the dosage form.

The HMG-CoA reductase composition may comprise a stabilizing metal or alkaline earth metal salt, additional excipients which are known as suitable agents in the art comprising combinations and concentrations as further described below. In a preferred embodiment, the HMG-CoA reductase composition contains conventional additional materials suitable for forming a tablet. Such excipients include a diluent, binder, and disintegrant. Antioxidants can also be incorporated into the HMG-CoA reductase inhibitor composition to prevent any oxidation of the drug compound. For example, antioxidants that could be used are butylated hydroxyanisole, sodium ascorbate, butylated hydroxytoluene, sodium metabisulfate, malic acid, citric acid and ascorbic acid.

In one HMG-CoA reductase inhibitor composition, the composition comprises a stabilizing agent, diluents, disintegrant, and surfactant. The basic excipient, calcium carbonate, has been found to chemically stabilize HMG-CoA reductase inhibitors, such as atorvastatin calcium. Microcrystalline cellulose and hydrous lactose are applied as suitable diluents. Croscarmellose sodium is present as a disintegrant. The non-ionic detergent Tween 80 is used as a surfactant. The composition also contains hydroxypropyl cellulose as binder selected from among several applicable substances such as, i.e., polyethylene glycol, polyvinylpyrrolidone, polyvinyl alcohol, hydroxymethylcellulose or hydroxypropylmethylcellulose. As antioxidants, reagents such as butylated hydroxyanisole, sodium ascorbate, ascorbic acid or others may optionally be incorporated in the composition. Magnesium stearate can be selected from a group including other substances such as stearic acid, palmitic acid, talc or similar lubricating compounds.

Other possible and supplemental ingredients such as preservatives, dryers, glidants, or colorants known as conventional by those skilled in the art may be included optionally in the HMG-CoA reductase inhibitor composition.

In one aspect, the HMG-CoA reductase inhibitor composition comprises the following concentration ranges of ingredients by weight: the HMG-CoA reductase inhibitor is in the range from about 1% to about 50%; calcium carbonate from about 5% to about 75%; microcrystalline cellulose from about 5% to about 75%; hydrous lactose from about 1% to about 80%; croscarmellose sodium from about 1% to about 15%; hydroxypropylcellulose from about 0.5% to about 6%; Tween 80 from about 0.1% to about 4%; magnesium stearate from about 0.25% to about 2%; and sodium ascorbate from about 0.0% to about 3%.

A more preferred HMG-CoA reductase inhibitor composition comprises the following approximate concentrations of ingredients by weight: about 13.9 wt% of the HMG-CoA reductase inhibitor atorvastatin hemicacium trihydrate; about 42.4 wt% of calcium carbonate; about 17.7 wt% microcrystalline cellulose; about 19.2 wt% pregelatanized starch; about 2.5 wt% hydroxypropyl cellulose; and about 0.5 wt% Tween 80.

The HMG-CoA reductase inhibitor composition may be formed by any conventional method for combining the HMG-CoA reductase inhibitor and excipients. Exemplary methods include wet and dry granulation. If wet granulation is used, a stabilizing agent such as calcium carbonate is preferably included to keep chemical degradation of the HMG-CoA reductase inhibitor at an acceptable level.

One exemplary method for forming the HMG-CoA reductase inhibitor composition comprises (a) milling an excess of the drug, (b) dissolving at least one binder additive in aqueous surfactant solution; (c) blending the milled drug

with at least one drug-stabilizing additive and at least one diluent additive with the drug-stabilizing additive and one half of a disintegrant additive in a rotary mixing vessel equipped with a chopping device; (d) granulating the blended drug ingredient mixture of step (c) with the surfactant/binder solution of step (b) in gradual increments in the chopper equipped mixing vessel; (e) drying the granulated drug mixture overnight at about 50°C; (f) sieving the dried granulated drug mixture; (g) tumble blending the sieved drug mixture with the remaining amount of the disintegrant additive; (h) mixing separately an aliquot of the drug mixture of step (g) with magnesium stearate, sieving same, and returning same to the drug mixture of step (g) and tumble blending the entire drug mixture.

In one embodiment, the CETP inhibitor composition and HMG-CoA reductase inhibitor composition are blended together and then compressed to form a tablet, caplet, pill, or other dosage forms formed by compression forces known in the art. In another embodiment, the CETP inhibitor composition and the HMG-CoA reductase inhibitor composition are blended together and filled into a capsule. In another embodiment, the CETP inhibitor composition and the HMG-CoA reductase inhibitor composition are blended together to form a powder dosage form. In another embodiment, the unitary dosage form is in the form of a kit comprising the CETP inhibitor composition and the HMG-CoA reductase inhibitor composition. The kit is designed such that the HMG-CoA reductase inhibitor and the solid amorphous dispersion are substantially separate.

Yet another embodiment of the unitary dosage form is a kit comprising two separate compositions: (1) one containing the solid amorphous dispersion comprising a CETP inhibitor and an acidic concentration-enhancing polymer, and (2) one containing the HMG-CoA reductase inhibitor. The kit is designed such that the HMG-CoA reductase inhibitor and the solid amorphous dispersion are substantially separate. The kit includes means for containing the separate compositions such as a divided bottle or a divided foil packet; however, the separate compositions may also be contained within a single, undivided container. Typically the kit includes directions for the administration of the separate components.

Further details of such unitary dosage forms are given in commonly assigned Provisional Patent Application No. 60/435,298, entitled "Dosage Forms Comprising a CETP Inhibitor and an HMG-CoA Reductase Inhibitor," filed December 20, 2002,

COATINGS

The unitary dosage form may optionally be coated with a conventional coating well known in the art. The coatings may be used to mask taste, improve appearance, facilitate swallowing of the dosage form, or to delay, sustain or otherwise control the release of the drug from the dosage form. Such coatings may be fabricated by any conventional means including fluidized bed coating, spray-coating, pan-coating and powder-coating using aqueous or organic solvents. Examples of suitable coating materials include sucrose, maltitol, cellulose acetate, ethyl cellulose, methylcellulose, sodium carboxymethyl cellulose, hydroxyethyl cellulose, hydroxypropyl cellulose, hydroxypropyl methylcellulose, polymethacrylates, polyacrylates, polyvinyl alcohol, polyvinyl pyrrolidone, cetyl alcohol, gelatin, maltodextrin, paraffin wax, microcrystalline wax, and Carnauba wax. Mixtures of polymers may also be used. Preferred coatings include the commercial aqueous coating formulations Surelease® and Opadry® available from Colorcon Inc. (West Point, Pennsylvania). Another exemplary commercial coating is Lustre Clear® from FMC Corp., located in Philadelphia, Pennsylvania.

In some cases, to avoid poor toleration or to avoid degradation, it is desired that drugs in the unitary dosage form not be released in the stomach. In these instances, the dosage form may also be overcoated with one or more pH-sensitive coating compositions, commonly referred to in the pharmaceutical arts as "enteric" coatings, by conventional procedures in order to delay the release of drug until it reaches the duodenum or small intestine. pH-sensitive polymers suitable as enteric coatings include those which are relatively insoluble and impermeable at the pH of the stomach, but which are more soluble or disintegrable or permeable at the pH of the duodenum and small intestine. Such pH-sensitive polymers include polyacrylamides, phthalate derivatives such as acid phthalate of carbohydrates, amylose acetate phthalate, cellulose acetate phthalate (CAP), other cellulose ester phthalates, cellulose ether phthalates, hydroxypropylcellulose phthalate (HPCP), hydroxypropyl ethylcellulose phthalate (HPECP), hydroxypropyl methylcellulose phthalate (HPMCP), HPMCAS, methylcellulose phthalate (MCP), polyvinyl acetate phthalate (PVAcP), polyvinyl acetate hydrogen phthalate, sodium CAP, starch acid phthalate, cellulose acetate trimellitate (CAT), styrene-maleic acid dibutyl phthalate copolymer, styrene-maleic acid/polyvinylacetate phthalate copolymer, styrene and maleic acid copolymers, polyacrylic acid derivatives such as acrylic acid and acrylic ester copolymers, polymethacrylic acid and esters thereof, polyacrylic and methacrylic acid copolymers, shellac and copolymers of vinyl acetate and crotonic acid.

A preferred group of pH-sensitive polymers includes CAP, PVAcP, HPMCP, HPMCAS, anionic acrylic copolymers of methacrylic acid and methylmethacrylate, and copolymers of acrylic acid and at least one acrylic acid ester.

To apply the pH-sensitive coating to the tablets, the pH-sensitive polymer is first dissolved in a suitable solvent to form a coating solution. Useful solvents for this purpose include ketones, such as acetone; alcohols, such as methanol, ethanol, isopropyl alcohol, n-propyl alcohol, and the various isomers of butanol; chlorinated hydrocarbons, such as methylene chloride; water; and mixtures of these solvents. The polymer may also be suspended in the solvent. The

coating solution may also comprise a latex of the pH-sensitive polymer suspended in an aqueous solution.

The coating solution may also contain one or more plasticizers, such as polyethylene glycols, triethyl citrate, propylene glycols, diethyl phthalate, dibutyl phthalate, castor oil, triacetin and others known in the art. The coating solution may also contain one or more emulsifiers, such as polysorbate-80. Coating is conducted in conventional fashion, typically by dipping, spray-coating, or pan-coating.

Where the coating is an acidic polymer, it may be desired to further protect the HMG-CoA reductase inhibitor from degradation from the coating material. In such instances, it may be desired to provide an inner coating surrounding the HMG-CoA reductase inhibitor composition onto which the exterior acidic coating may be applied. Other conventional techniques may be used to prevent the coating from degrading the HMG-CoA reductase inhibitor.

The coating solution may also contain a base or buffer, such as those discussed above. Use of a base or buffer will ensure the pH of the coating solution is not so low as to increase chemical degradation of the HMG-CoA reductase inhibitor. Use of a base or buffer may also be used to minimize reaction of the coating formulation with other excipients in the dosage form.

The unitary dosage forms of the present invention may be used to treat any condition, which is subject to treatment by administering a CETP inhibitor and an HMG-CoA reductase inhibitor, as disclosed in commonly assigned, copending U.S. Patent Application No. 2002/0035125A1.

In one aspect, the unitary dosage forms of the present invention are used for antiatherosclerotic treatment.

In another aspect, the unitary dosage forms of the present invention are used for slowing and/or arresting the progression of atherosclerotic plaques.

In another aspect, the unitary dosage forms of the present invention are used for slowing the progression of atherosclerotic plaques in coronary arteries.

In another aspect, the unitary dosage forms of the present invention are used for slowing the progression of atherosclerotic plaques in carotid arteries.

In another aspect, the unitary dosage forms of the present invention are used for slowing the progression of atherosclerotic plaques in the peripheral arterial system.

In another aspect, the unitary dosage forms of the present invention, when used for treatment of atherosclerosis, causes the regression of atherosclerotic plaques.

In another aspect, the unitary dosage forms of the present invention are used for regression of atherosclerotic plaques in coronary arteries.

In another aspect, the unitary dosage forms of the present invention are used for regression of atherosclerotic plaques in carotid arteries.

In another aspect, the unitary dosage forms of the present invention are used for regression of atherosclerotic plaques in the peripheral arterial system.

In another aspect, the unitary dosage forms of the present invention are used for HDL elevation treatment and antihyperlipidemic treatment (including LDL lowering).

In another aspect, the unitary dosage forms of the present invention are used for antianginal treatment.

In another aspect, the unitary dosage forms of the present invention are used for cardiac risk management.

Other features and embodiments of the invention will become apparent from the following examples, which are given for illustration of the invention, rather than for limiting its intended scope.

## EXAMPLES

### Example 1

Crystalline atorvastatin calcium was combined with a solid amorphous dispersion containing a CETP inhibitor and a neutralized acidic polymer, and stored at 50°C and 75% relative humidity for 3 weeks. The stability of atorvastatin was improved relative to a control composition containing an acidic polymer.

The following process was used to form a solid amorphous dispersion containing 25 wt% of the CETP inhibitor torcetrapib, also known as [2R,4S]-4-[(3,5-bis-trifluoromethyl-benzyl)-methoxycarbonyl-amino]-2-ethyl-6-trifluoromethyl-3,4-dihydro-2H-quinoline-1-carboxylic acid ethyl ester, (torcetrapib) and 75 wt% hydroxypropylmethyl cellulose acetate succinate (HPMCAS)(MF grade, available from Shin Etsu, located in Tokyo, Japan) wherein greater than 95% of the acidic succinate substituents had been neutralized with potassium hydroxide. First, a spray solution was formed containing 1500 mg HPMCAS (an acidic polymer) in 45 g methanol with 82.3 mg potassium hydroxide (a sufficient amount to completely neutralize the acidic groups on the polymer). Next, 500 mg torcetrapib and about 3 mL water were dissolved therein. The solution was pumped into a "mini" spray-drying apparatus via a Cole Parmer 74900 series rate-controlling syringe pump at a rate of 1.3 mL/min. The solution was sprayed using a Spraying Systems Co. two-fluid nozzle, model number SU1A, with nitrogen as the atomizing gas. The nitrogen was pressurized and heated to a temperature of 70°C at a flow rate of 1.0 scfm. The solution was sprayed from the top of an 11-cm diameter stainless steel

chamber. The resulting solid amorphous dispersion was collected on Whatman® filter paper, dried under vacuum, and stored in a dessicator. After drying, the solid amorphous dispersion contained 25 wt% torcetrapib and 75 wt% neutralized HPMCAS-MF.

The spray-dried solid amorphous dispersion was evaluated in an *in vitro* dissolution test using a microcentrifuge method. In this test, 7.2 mg of the spray-dried solid amorphous dispersion was placed into a microcentrifuge tube. The tube was placed in a 37°C sonicating bath, and 1.8 mL phosphate buffered saline (PBS) at pH 6.5 and 290 mOsm/kg was added, resulting in a torcetrapib concentration of 1000 μg/mL if all of the drug had dissolved. The sample was quickly mixed using a vortex mixer for about 60 seconds. The sample was centrifuged at 13,000 G at 37°C for 1 minute. The resulting supernatant solution was then sampled and diluted 1:6 (by volume) with methanol and then analyzed by high-performance liquid chromatography (HPLC). The contents of the tube was mixed on the vortex mixer and allowed to stand undisturbed at 37°C until the next sample was taken. Samples were collected at 4, 10, 20, 40, 90, and 1200 minutes. The concentrations of drug obtained in these samples are shown in Table 1, which represent the average of duplicate tests.

As a control, an *in vitro* dissolution test was performed using the procedures described above except that 1.8 mg of crystalline drug was used. The concentrations of drug obtained in *in vitro* dissolution tests are shown in Table 1.

Table 1

| Sample | Time (min) | Torcetrapib Concentration (μg/mL) | AUC (min-μg/mL) |
|---|---|---|---|
| Solid Amorphous Dispersion using the neutralized HPMCAS-MG/$K^+$ | 0 | 0 | 0 |
| | 4 | 190 | 400 |
| | 10 | 189 | 1500 |
| | 20 | 165 | 3300 |
| | 40 | 154 | 6500 |
| | 90 | 119 | 13,300 |
| | 1200 | 17 | 88,800 |
| Crystalline Drug | 0 | 0 | 0 |
| | 4 | <1 | <2 |
| | 10 | <1 | <8 |
| | 20 | <1 | <18 |
| | 40 | <1 | <38 |
| | 90 | <1 | <88 |
| | 1200 | <1 | <1,200 |

The results of these dissolution tests are summarized in Table 2, which shows the maximum concentration of torcetrapib in solution during the first 90 minutes of the test ($C_{max,90}$), the area under the aqueous concentration versus time curve after 90 minutes ($AUC_{90}$), and the concentration at 1200 minutes ($C_{1200}$).

Table 2

| Sample | Concentration-Enhancing Polymer | Torcetrapib Conc. in the Dispersion (wt%) | Receptor Solution | $C_{max,90}$ (μg/mL) | AUC90 (min-μg/mL) |
|---|---|---|---|---|---|
| Solid Amorphous Dispersion | HPMCAS-MG Neutralized with $K^+$ | 25 | PBS | 190 | 13,300 |
| Crystalline Drug | None | NA | PBS | <1 | <88 |

The results summarized in Table 2 show that the solid amorphous dispersion provided concentration enhancement relative to crystalline drug. The solid amorphous dispersion provided a $C_{max,90}$ value that was greater than 190-fold that of the crystalline drug, and an $AUC_{90}$ value that was greater than 151-fold that of the crystalline drug.

Example 1 consisted of a mixture of crystalline atorvastatin (14.3 wt%) and the CETP inhibitor dispersion with neutralized polymer (85.7 wt%). To form the mixture of Example 1, 42.9 mg of crystalline atorvastatin and 257.1 mg of the solid amorphous dispersion were combined and blended for 20 minutes using a Turbula mixer. The mixture was

slugged using a Carver press at 500 psi, and then milled using a mortar and pestle. The granules were screened using a #20 sieve.

Control 1 consisted of a mixture of crystalline atorvastatin (14.3 wt%) and the CETP inhibitor dispersion with un-neutralized acidic polymer (85.7 wt%). The solid amorphous dispersion for Control 1 was made by forming a spray solution containing 25 g torcetrapib, 75 g HPMCAS (MG grade, available from Shin Etsu, located in Tokyo, Japan), and 900 g acetone. The spray solution was pumped using a high-pressure pump (Zenith Z-Drive 2000 High-Pressure Gear Pump) to a spray drier (Niro type XP Portable Spray-Dryer with a Liquid-Feed Process Vessel [PSD-1]) equipped with a pressure atomizer (Spraying Systems Pressure Nozzle and Body (SK 79-16)). The PSD-1 was equipped with a 9-inch chamber extension. The spray drier was also equipped with a diffuser plate having a 1% open area. The nozzle sat flush with the diffuser plate during operation. The spray solution was pumped to the spray drier at about 185 g/min, with an atomization pressure of about 280 psi. Drying gas (nitrogen) was circulated through the diffuser plate at an inlet temperature of about 98°C. The evaporated solvent and wet drying gas exited the spray drier at a temperature of about 29°C. The spray-dried solid amorphous dispersion formed by this process was collected in a cyclone, and had a bulk specific volume of 4.5 $cm^3$/gm. The solid amorphous dispersion was post-dried using a Gruenberg single-pass convection tray dryer operating at 40°C for about 16 hours. To form the mixture of Control 1, 42.9 mg of crystalline atorvastatin and 257.1 mg of the torcetrapid solid amoprhous dispersion with un-neutralized polymer were combined, then blended, slugged, milled, and sieved as described for Example 1.

Example 1 and Control 1 were stored at 50°C and 75% relative humidity for 3 weeks to increase the rate of chemical and physical changes occurring in the materials in order to simulate a longer storage interval in a typical storage environment. Following storage, samples were analyzed for atorvastatin purity using HPLC. To analyze the samples by HPLC, about 0.4 mg/mL atorvastatin in the mixture was added to a dissolving solvent. The dissolving solvent was made by combining 150 mLs 50mM ammonium acetate (pH 7.0), 600 mLs acetonitrile, and 250 mLs methanol. Mobile phase A was made by adding 3 mLs acetic acid to 530 mLs water, adjusting to pH 4.0 with ammonium hydroxide, then adding 270 mLs acetonitrile and 200 mLs tetrahydrofuran. Mobile phase B was made by adding 1 mL acetic acid to 100 mLs water, adding half of the amount of ammonium hydroxide used to adjust Mobile phase A, then adding 700 mLs acetonitrile and 200 mLs tetrahydrofuran. The samples were analyzed using a Waters Spherisorb ODS2 column, with a solvent flow rate of 1.5 mL/min. Table 3 shows the solvent gradient used.

Table 3

| Time | %A | %B |
|---|---|---|
| 0 | 100 | 0 |
| 15 | 100 | 0 |
| 35 | 0 | 100 |
| 50 | 0 | 100 |
| 51 | 100 | 0 |
| 60 | 100 | 0 |

The UV absorbance of atorvastatin and atorvastatin impurities were measured at a wavelength of 244 nm. The atorvastatin lactone impurity eluting after about 10 minutes was chosen as the basis for comparison. All peak areas were added and the lactone impurity as percent of total peak area was calculated to give the degree of degradation. Results are shown below in Table 4.

Table 4

| Sample | Degree of Degradation (wt%) |
|---|---|
| Example 1 | 0.06 |
| Control 1 | 0.49 |

The results from Table 4 show that the atorvastatin in the sample of Control 1 (atorvastatin mixed with the CETPI/un-neutralized acidic polymer dispersion) contained 0.49 wt% lactone impurity. Example 1 shows that a composition containing a CETP inhibitor solid amorphous dispersion comprising a neutralized acidic polymer provided improved stability of atorvastatin relative to a composition containing a dispersion comprising an acidic polymer.

A relative degree of improvement in chemical stability was determined by taking the ratio of the degree of

degradation of the drug in the control composition and the degree of degradation of the drug in the composition of Example 1. For Example 1, where the degree of degradation of atorvastatin is 0.06 wt%, and the degree of degradation of Control 1 is 0.49 wt%, the relative degree of improvement is 0.49 wt%/0.06 wt%, or 8.17.

Example 2 (not encompassed by the scope of claim 1)

Crystalline atorvastatin was combined with an amorphous dispersion containing a CETP inhibitor and a neutral polymer, and stored at 50°C and 75% relative humidity for 3 weeks. The stability of atorvastatin was improved relative to a control composition containing an acidic polymer.

The following process was used to form a solid amorphous dispersion containing 25 wt% torcetrapib and 75 wt% of the neutral polymer hydroxypropyl methyl cellulose (HPMC). First, a spray solution was formed containing 1500 mg HPMC (E3 Prem Methocel® obtained from Dow Chemical Co., Midland, Michigan) in 48 g methanol. Next, 500 mg torcetrapib and about 2 mLs water were added. The solution was pumped into a "mini" spray-drying apparatus and spray-dried as described for Example 1. After drying, the resulting amorphous dispersion contained 25 wt% torcetrapib and 75 wt% HPMC.

The spray-dried solid amorphous dispersion was evaluated in an *in vitro* dissolution test using a microcentrifuge method as described in Example 1. The concentrations of drug obtained in these samples are shown in Table 5, which represent the average of duplicate tests.

Table 5

| Sample | Time (min) | Torcetrapib Concentration (μg/mL) | AUC (min-μg/mL) |
|---|---|---|---|
| Solid Amorphous Dispersion using the neutral polymer HPMC | 0 | 0 | 0 |
| | 4 | 87 | 200 |
| | 10 | 21 | 500 |
| | 20 | 12 | 700 |
| | 40 | 9 | 900 |
| | 90 | 11 | 1400 |
| | 1200 | 5 | 10,100 |

The results of these dissolution tests are summarized in Table 6, which shows the maximum concentration of torcetrapib in solution during the first 90 minutes of the test ($C_{max,90}$), the area under the aqueous concentration versus time curve after 90 minutes ($AUC_{90}$), and the concentration at 1200 minutes ($C_{1200}$). The results for the crystalline drug are included for comparison.

Table 6

| Sample | Concentration-Enhancing Polymer | Torcetrapib Conc. in the Dispersion (wt%) | Receptor Solution | $C_{max,90}$ (μg/mL) | AUC90 (min-μg/mL) |
|---|---|---|---|---|---|
| Solid Amorphous Dispersion | HPMC | 25 | PBS | 87 | 1,400 |
| Crystalline Drug | None | NA | PBS | <1 | <88 |

The results summarized in Table 6 show that the solid amorphous dispersion provided concentration enhancement relative to crystalline drug. The solid amorphous dispersion provided a $C_{max,90}$ value that was greater than 87-fold that of the crystalline drug, and an $AUC_{90}$ value that was greater than 15-fold that of the crystalline drug.

Example 2 consisted of a mixture of crystalline atorvastatin (14.3 wt%) and the CETP inhibitor dispersion with neutral polymer (85.7 wt%). To form the mixture of Example 2, 42.9 mg of crystalline atorvastatin and 257.1 mg of the solid amorphous dispersion above were combined, then blended, slugged, milled, and sieved as described for Example 1.

Example 2 was stored at 50°C and 75% relative humidity for 3 weeks, then analyzed for atorvastatin purity using HPLC, as described for Example 1 and Control 1. All impurity peak areas were added and the lactone impurity as percent of total peak area was calculated to give the degree of degradation. Results are shown below in Table 7. Control 1 is shown again for comparison.

Table 7

| Sample | Degree of Degradation (wt%) |
|---|---|
| Example 2 | 0.13 |
| Control 1 | 0.49 |

The atorvastatin in the sample of Example 2 contained 0.13 wt% lactone impurity after storage. The atorvastatin in the sample of Control 1 (atorvastatin mixed with the CETP inhibitor/HPMCAS dispersion) contained 0.49 wt% lactone impurity after storage. Example 2 shows that use of a CETP inhibitor dispersion comprising a neutral polymer provided improved atorvastatin stability relative to a composition comprising a dispersion containing an acidic polymer. For Example 2, where the degree of degradation of atorvastatin was 0.13 wt%, and the degree of degradation of Control 1 was 0.49 wt%, the relative degree of improvement was 0.49 wt%/0.13 wt%, or 3.8.

Example 3

This example demonstrates neutralizing an acidic concentration-enhancing polymer by adding base following formation of the solid amorphous dispersion.

Example 3 consisted of a mixture of crystalline atorvastatin (13.9 wt%), magnesium oxide (3.0 wt%), and the CETP inhibitor dispersion with acidic polymer HPMCAS described for Control 1 (83.1 wt%). To form the mixture of Example 3, 42.9 mg of crystalline atorvastatin, 9.4 mg of magnesium oxide, and 257.1 mg of the solid amorphous dispersion containing 25 wt% torcetrapib and 75 wt% HPMCAS were combined, then blended, slugged, milled, and sieved as described for Example 1. Example 3 was stored at 50°C and 75% relative humidity for 3 weeks, then analyzed for atorvastatin purity using HPLC, as described for Example 1 and Control 1. Results are shown below in Table 8.

Table 8

| Sample | Degree of Degradation (wt%) |
|---|---|
| Example 3 | 0.19 |
| Control 1 | 0.49 |

The results from Table 8 show that the atorvastatin in the sample of Example 3 contains 0.19 wt% lactone impurity. The relative degree of improvement for Example 3 is 2.6 relative to Control 1.

The terms and expressions which have been employed in the foregoing specification are used therein as terms of description and not of limitation, and there is no intention, in the use of such terms and expressions, of excluding equivalents of the features shown and described or portions thereof, it being recognized that the scope of the invention is defined and limited only by the claims which follow.

## Claims

**1.** A unitary dosage form comprising:

(a) a solid amorphous dispersion comprising a cholesteryl ester transfer protein inhibitor and a concentration-enhancing polymer; and
(b) an HMG-CoA reductase inhibitor;

wherein said concentration-enhancing polymer is a neutralized acidic polymer in which at least 90% of the acidic moieties have been neutralized;
and wherein the cholesteryl ester transfer protein inhibitor is (2R)-3-[[3-(4-chloro-3-ethylphenoxy)phenyl][[3-(1,1,2.2-tetrafluoroethoxy)phenyl]-methyl]amino]-1,1,1-trifluoro-2-propanol.

**2.** The unitary dosage form of claim 1 wherein said neutralized acidic polymer is a neutralized form of a polymer selected from the group consisting of hydroxypropyl methyl cellulose acetate succinate, hydroxypropyl methyl cellulose succinate, hydroxypropyl cellulose acetate succinate, hydroxyethyl methyl cellulose succinate, hydroxyethyl cellulose acetate succinate, hydroxypropyl methyl cellulose phthalate, hydroxyethyl methyl cellulose acetate succinate, hydroxyethyl methyl cellulose acetate phthalate, cellulose acetate phthalate, methyl cellulose acetate phthalate, ethyl cellulose acetate phthalate, hydroxypropyl cellulose acetate phthalate, hydroxypropyl methyl cellulose acetate phthalate, hydroxypropyl cellulose acetate phthalate succinate, hydroxypropyl methyl cellulose acetate succinate phthalate, hydroxypropyl methyl cellulose succinate phthalate, cellulose propionate phthalate, hydroxypropyl cellulose butyrate phthalate, cellulose acetate trimellitate, methyl cellulose acetate trimellitate, ethyl cellulose acetate trimellitate, hydroxypropyl cellulose acetate trimellitate, hydroxypropyl methyl cellulose acetate trimellitate, hydroxypropyl cellulose acetate trimellitate succinate, cellulose propionate trimellitate, cellulose butyrate trimellitate, cellulose acetate terephthalate, cellulose acetate isophthalate, cellulose acetate pyridinedicarboxylate, salicylic acid cellulose acetate, hydroxypropyl salicylic acid cellulose acetate, ethylbenzoic acid cellulose acetate, hydroxypropyl ethylbenzoic acid cellulose acetate, ethyl phthalic acid cellulose acetate, ethyl nicotinic acid cellulose acetate, ethyl picolinic acid cellulose acetate, carboxymethyl ethyl cellulose, carboxylic acid functionalized vinyl polymers, carboxylic acid functionalized polymethacrylates, carboxylic acid functionalized polyacrylates, and mixtures thereof.

**3.** The unitary dosage form of claim 1 further comprising an excipient selected from the group consisting of a base and a buffer.

**4.** The unitary dosage form of claim 1 wherein said HMG-CoA reductase inhibitor is selected from the group consisting of fluvastatin, lovastatin, pravastatin, atorvastatin, simvastatin, rivastatin, mevastatin, velostatin, compactin, dalvastatin, fluindostatin, rosuvastatin, pitivastatin, dihydrocompactin, cerivastatin, and pharmaceutically acceptable forms thereof.

**5.** The unitary dosage form of any one of claim 4 wherein said HMG-CoA reductase inhibitor is selected from the group consisting of atorvastatin and pharmaceutically acceptable forms thereof.

**6.** The unitary dosage form of claim 1 wherein said dosage form is selected from the group consisting of a tablet, caplet, pill, capsule, powder, and a kit comprising one or more tablets, caplets, pills, capsules, sachets, powders, or solutions intended to be taken together.

**7.** A method for forming a unitary dosage form comprising:

(a) forming a solid amorphous dispersion comprising a cholesteryl ester transfer protein inhibitor and a concentration-enhancing polymer, wherein the cholesteryl ester transfer protein inhibitor is (2R)-3-{[3-(4-chloro-3-ethylphenoxy)phenyl] [[3-(1,1,2,2-tetrafluoroethoxy)phenyl]-methyl]amino}-1,1,1-trifluoro-2-propanol; and
(b) combining said solid amorphous dispersion with an HMG-CoA reductase inhibitor to form said unitary dosage form;

wherein said concentration-enhancing polymer is a neutralized acidic polymer in which at least 90% of the acidic moieties have been neutralized and wherein said acidic polymer and said cholesteryl ester transfer protein inhibitor are formed into said solid amorphous dispersion, and said solid amorphous dispersion is then combined with at least one of a base and a buffer to form said neutralized acidic polymer.

**Patentansprüche**

**1.** Einheitsdosierungsform, umfassend:

(a) eine feste amorphe Dispersion, umfassend einen Cholesterylestertransferproteininhibitor und ein die Konzentration erhöhendes Polymer; und
(b) einen HMG-CoA-Reduktase-Inhibitor;

wobei das die Konzentration erhöhende Polymer ein neutralisiertes saures Polymer ist, in welchem wenigstens 90% der sauren Gruppierungen neutralisiert wurden; und wobei der Cholesterylestertransferproteininhibitor (2R)-3-{[3-(4-Chlor-3-ethylphenoxy)phenyl][[3-(1,1,2,2-tetrafluorethoxy)phenyl]methyl]amino}-1,1,1-trifluor-2-propanol ist.

**2.** Einheitsdosierungsform nach Anspruch 1, wobei das neutralisierte saure Polymer eine neutralisierte Form eines Polymers ist, ausgewählt aus der Gruppe, bestehend aus Hydroxypropylmethylcelluloseacetatsuccinat, Hydroxypropylmethylcellulosesuccinat, Hydroxypropylcelluloseacetatsuccinat, Hydroxyethylmethylcellulosesuccinat,

Hydroxyethylcelluloseacetatsuccinat, Hydroxypropylmethylcellulosephthalat, Hydroxyethylmethylcelluloseacetatsuccinat, Hydroxyethylmethylcelluloseacetatphthalat, Celluloseacetatphthalat, Methylcelluloseacetatphthalat, Ethylcelluloseacetatphthalat, Hydroxypropylcelluloseacetatphthalat, Hydroxypropylmethylcelluloseacetatphthalat, Hydroxypropylcelluloseacetatphthalatsuccinat, Hydroxypropylmethylcelluloseacetatsuccinatphthalat, Hydroxypropylmethylcellulosesuccinatphthalat, Cellulosepropionatphthalat, Hydroxypropylcellulosebutyratphthalat, Celluloseacetattrimellitat, Methylcelluloseacetattrimellitat, Ethylcelluloseacetattrimellitat, Hydroxypropylcelluloseacetattrimellitat, Hydroxypropylmethylcelluloseacetattrimellitat, Hydroxypropylcelluloseacetattrimellitatsuccinat, Cellulosepropionattrimellitat, Cellulosebutyrattrimellitat, Celluloseacetatterephthalat, Celluloseacetatisophthalat, Celluloseacetatpyridindicarboxylat, Salicylsäurecelluloseacetat, Hydroxypropylsalicylsäurecelluloseacetat, Ethylbenzoesäurecelluloseacetat, Hydroxypropylethylbenzoesäurecelluloseacetat, Ethylphthalsäure, Celluloseacetat, Ethylnicotinsäurecelluloseacetat, Ethylpicolinsäurecelluloseacetat, Carboxymethylethylcellulose, Carbonsäure-funktionalisierten Vinylpolymeren, Carbonsäure-funktionalisierten Polymethacrylaten, Carbonsäure-funktionalisierten Polyacrylaten und Gemischen davon.

**3.** Einheitsdosierungsform nach Anspruch 1, die außerdem ein Exzipiens umfasst, das aus der Gruppe, bestehend aus einer Base und einem Puffer, ausgewählt ist.

**4.** Einheitsdosierungsform nach Anspruch 1, wobei der HMG-CoA-Reduktase-Inhibitor aus der Gruppe, bestehend aus Fluvastatin, Lovastatin, Pravastatin, Atorvastatin, Simvastatin, Rivastatin, Mevastatin, Velostatin, Compactin, Dalvastatin, Fluindostatin, Rosuvastatin, Pitivastatin, Dihydrocompactin, Cerivastatin und pharmazeutisch verträglichen Formen davon, ausgewählt ist.

**5.** Einheitsdosierungsform nach Anspruch 4, wobei der HMG-CoA-Reduktase-Inhibitor aus der Gruppe, bestehend aus Atorvastatin und pharmazeutisch verträglichen Formen davon, ausgewählt ist.

**6.** Einheitsdosierungsform nach Anspruch 1, wobei die Dosierungsform aus der Gruppe, bestehend aus einer Tablette, einem Caplet, einer Pille, einer Kapsel, einem Pulver und einem Kit, der eine oder mehrere Tablette(n), Caplet(s), Pille(n), Kapsel(n), Sachet(s), Pulver oder Lösung(en) umfasst, die dazu bestimmt sind, zusammengenommen zu werden, ausgewählt ist.

**7.** Verfahren zur Bildung einer Einheitsdosierungsform, umfassend:

(a) Bilden einer festen amorphen Dispersion, die einen Cholesterylestertransferproteininhibitor und ein die Konzentration erhöhendes Polymer umfasst, wobei der Cholesterylestertransferproteininhibitor (2R)-3-{[3-(4-Chlor-3-ethylphenoxy)phenyl][[3-(1,1,2,2-tetrafluorethoxy)phenyl]-methyl]amino}-1,1,1-trifluor-2-propanol ist, und
(b) Kombinieren der festen amorphen Dispersion mit einem HMG-CoA-Reduktase-Inhibitor unter Bildung der Einheitsdosierungsform;

wobei das die Konzentration erhöhende Polymer ein neutralisiertes saures Polymer ist, in welchem wenigstens 90% der sauren Gruppierungen neutralisiert wurden und wobei das saure Polymer und der Cholesterylestertransferproteininhibitor zu der festen amorphen Dispersion geformt werden und wobei die feste amorphe Dispersion dann mit wenigstens einem einer Base und eines Puffers kombiniert wird, um das neutralisierte saure Polymer zu bilden.

## Revendications

**1.** Forme posologique unitaire comprenant :

(a) une dispersion amorphe solide comprenant un inhibiteur de la protéine de transfert d'ester de cholestéryle et un polymère augmentant la concentration ; et
(b) un inhibiteur de HMG-CoA-réductase ;

dans laquelle ledit polymère augmentant la concentration est un polymère acide neutralisé dans lequel au moins 90 % des groupements acides ont été neutralisés ;
et dans laquelle l'inhibiteur de la protéine de transfert d'ester de cholestéryle est le (2R)-3-[[3-(4-chloro-3-éthylphénoxy)phényl][[3-(1,1,2,2-tétrafluoréthoxy)-phényl]-méthyl]amino]-1,1,1-trifluoro-2-propanol.

2. Forme posologique unitaire suivant la revendication 1, dans laquelle ledit polymère acide neutralisé est une forme neutralisée d'un polymère choisi dans le groupe consistant en l'acétosuccinate d'hydroxypropylméthylcellulose, le succinate d'hydroxypropylméthylcellulose, l'acétosuccinate d'hydroxypropylcellulose, le succinate d'hydroxyéthylméthylcellulose, l'acétosuccinate d'hydroxyéthylcellulose, le phtalate d'hydroxypropylméthylcellulose, l'acétosuccinate d'hydroxy-éthylméthylcellulose, l'acétophtalate d'hydroxyéthylméthyl-cellulose, l'acétophtalate de cellulose, l'acétophtalate de méthylcellulose, l'acétophtalate d'éthylcellulose, l'acétophtalate d'hydroxypropylcellulose, l'acétophtalate d'hydroxypropylméthylcellulose, l'acétophtalate-succinate d'hydroxypropylcellulose, l'acétosuccinate-phtalate d'hydroxy-propylméthylcellulose, le succinate-phtalate d'hydroxypropyl-méthylcellulose, le propionate-phtalate de cellulose, le butyrate-phtalate d'hydroxypropylcellulose, l'acétotrimellitate de cellulose, l'acétotrimellitate de méthylcellulose, l'acétotrimellitate d'éthylcellulose, l'acétotrimellitate d'hydroxypropylcellulose, l'acétotrimellitate d'hydroxypropyl-méthylcellulose, l'acétotrimellitate-succinate d'hydroxypropylcellulose, le propionate-trimellitate de cellulose, le butyrate-trimellitate de cellulose, l'acéto-téréphtalate de cellulose, l'acéto-isophtalate de cellulose, l'acétopyridinedicarboxylate de cellulose, l'acétate d'acide salicylique-cellulose, l'acétate d'acide hydroxypropyl-salicylique-cellulose, l'acétate d'acide d'éthylbenzoïque-cellulose, l'acétate d'acide hydroxypropyléthylbenzoïque-cellulose, l'acétate d'acide éthylphtalique-cellulose, l'acétate d'acide éthylnicotinique-cellulose, l'acétate d'acide éthylpicolinique-cellulose, la carboxyméthyléthylcellulose, des polymères vinyliques à fonctionnalisation acide carboxylique, des polyméthacrylates à fonctionnalisation acide carboxylique, des polyacrylates à fonctionnalisation acide carboxylique et leurs mélanges.

3. Forme posologique unitaire suivant la revendication 1, comprenant en outre un excipient choisi dans le groupe consistant en une base et un tampon.

4. Forme posologique unitaire suivant la revendication 1, dans laquelle ledit inhibiteur de HMG-CoA-réductase est choisi dans le groupe consistant en la fluvastatine, la lovastatine, la pravastatine, l'atorvastatine, la simvastatine, la rivastatine, la mévastatine, la vélostatine, la compactine, la dalvastatine, la fluindostatine, la rosuvastatine, la pitivastatine, la dihydrocompactine, la cérivastatine et leurs formes pharmaceutiquement acceptables.

5. Forme posologique unitaire suivant l'une quelconque de la revendication 4, dans laquelle ledit inhibiteur de HMB-CoA-réductase est choisi dans le groupe consistant en l'atorvastatine et ses formes pharmaceutiquement acceptables.

6. Forme posologique unitaire suivant la revendication 1, ladite forme posologique étant choisie dans le groupe consistant en un comprimé, un caplet, une pilule, une capsule, une poudre et un kit comprenant un ou plusieurs comprimés ou caplets, une ou plusieurs pilules ou capsules, un ou plusieurs sachets, une ou plusieurs poudres ou solutions, destinés à être pris ensemble.

7. Procédé pour former une forme posologique unitaire, comprenant les étapes consistant à :

(a) former une dispersion amorphe solide comprenant un inhibiteur de la protéine de transfert d'ester de cholestéryle et un polymère augmentant la concentration, ledit inhibiteur de la protéine de transfert d'ester de cholestéryle étant le (2R)-3-{[3-(4-chloro-3-éthylphénoxy)phényl][[3-(1,1,2,2-tétrafluoréthoxy)phényl]-méthyl]amino}-1,1,1-trifluoro-2-propanol ; et
(b) combiner ladite dispersion amorphe solide avec un inhibiteur de HMG-CoA-réductase pour former ladite forme posologique unitaire ;

dans lequel ledit polymère augmentant la concentration est un polymère acide neutralisé dans lequel au moins 90 % des groupements acides ont été neutralisés et dans lequel ledit polymère acide et ledit inhibiteur de la protéine de transfert d'ester de cholestéryle sont mis sous forme de ladite dispersion amorphe solide, et ladite dispersion amorphe solide est ensuite combinée avec au moins un des agents consistant en une base et un tampon pour former ledit polymère acide neutralisé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0211710 A2
- WO 0213797 A2
- US 6310075 B1, DeNinno
- US 6197786 B1, DeNinno
- WO 0038722 A
- US 5932587 A, Schmeck
- US 6126971 A
- WO 0018724 A
- US 5456923 A
- US 5939099 A
- US 5340591 A
- US 4673564 A
- US 5707646 A
- US 4894235 A
- US 353986 P
- US 354080 P
- US 918127 A
- US 10066091 A
- US 4231938 A
- US 4294926 A
- US 4319039 A
- US 4444784 A
- US 4450171 A
- US 4820850 A
- US 4916239 A
- US 4346227 A
- US 4537859 A
- US 4410629 A
- US 5030447 A
- US 5180589 A
- US 4448979 A
- US 5354772 A
- US 4911165 A
- US 4739073 A
- US 4929437 A
- US 5189164 A
- US 5118853 A
- US 5290946 A
- US 5356896 A
- US 5273995 A
- US 4681893 A
- US 5489691 A
- US 5342952 A
- US 5177080 A
- EP 491226 A
- US 5260440 A
- US RE37314 E
- EP 521471 A
- US 3983140 A
- US 5217992 A
- US 5196440 A
- US 5189180 A
- US 5166364 A
- US 5157134 A
- US 5110940 A
- US 5106992 A
- US 5099035 A
- US 5081136 A
- US 5049696 A
- US 5049577 A
- US 5025017 A
- US 5011947 A
- US 5010105 A
- US 4970221 A
- US 4940800 A
- US 4866058 A
- US 4686237 A
- US 4647576 A
- EP 0142146 A2
- EP 0221025 A1
- WO 8603488 A
- WO 8607054 A
- WO 9706802 A
- US 60435298 B
- US 20020035125 A1


### Non-patent literature cited in the description


- Remington: The Science and Practice of Pharmacy. 2000
- Pharmacokinetics: Processes and Mathematics. **WELLING.** ACS Monograph. Amer. Chem. Soc, 1986, vol. 185
- Perry's Chemical Engineers' Handbook. 1984, 20-5420-57
- **MARSHALL.** Atomization and Spray-Drying. *Chem. Eng. Prog. Monogr. Series 2,* 1954, vol. 50
- **MASTERS.** Spray Drying Handbook. 1985